(19)

Europäisches Patentamt

European Patent Office

Office européen des brevets

(11) **EP 4 643 866 A1**

(12) **EUROPEAN PATENT APPLICATION**
published in accordance with Art. 153(4) EPC

(43) Date of publication:
05.11.2025 Bulletin 2025/45

(21) Application number: 23912206.2

(22) Date of filing: 26.12.2023

(51) International Patent Classification (IPC):
*A61K 36/725* (2006.01)   *A23L 33/105* (2016.01)
*A61K 36/882* (2006.01)   *A61P 25/00* (2006.01)
*A61P 25/28* (2006.01)    *A61P 43/00* (2006.01)

(52) Cooperative Patent Classification (CPC):
A23L 33/105; A61K 36/725; A61K 36/882;
A61P 25/00; A61P 25/28; A61P 43/00

(86) International application number:
PCT/JP2023/046811

(87) International publication number:
WO 2024/143428 (04.07.2024 Gazette 2024/27)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 27.12.2022 JP 2022210257

(71) Applicant: Cerebro Pharma Inc.
Osaka-shi, Osaka 541-0058 (JP)

(72) Inventors:
• TOMIYAMA, Takami
  Osaka-shi, Osaka 541-0058 (JP)

• UMEDA, Tomohiro
  Osaka-shi, Osaka 541-0058 (JP)
• YAMANA, Kei
  Osaka-shi, Osaka 530-0005 (JP)
• NAKAJIMA, Ryota
  Osaka-shi, Osaka 530-0005 (JP)
• NAKATA, Kunio
  Osaka-shi, Osaka 530-0005 (JP)

(74) Representative: HGF
HGF Limited
1 City Walk
Leeds LS11 9DX (GB)

(54) **AGENT AND METHOD FOR MAINTAINING OR IMPROVING CEREBRAL FUNCTION USING HERBAL MEDICINE**

(57)    As a new means for maintaining or improving cognitive function, provided is an agent that is for maintaining or improving cerebral function and that contains an herbal medicine selected from a stem portion of Japanese sweet flag and a jujube seed. Here, said jujube seed is chosen from a crushed material of the same or an extraction residue of the crushed material, and said stem portion of Japanese sweet flag is chosen from a crushed material of the same, an extract of the crushed material, or an extraction residue of the crushed material.

Fig. 1

EP 4 643 866 A1

## Description

## TECHNICAL FIELD

The present invention relates to agents and methods using herbal medicines for maintaining or improving cerebral functions, promoting nerve cell repair or inducing neurogenesis, removing dementia-causing proteins that accumulate in the brain, and treating or preventing neurodegenerative diseases, as well as food and pharmaceutical products containing such agents.

## BACKGROUND ART

As societies are aging and adopting more Western lifestyles and eating habits, the number of dementia patients is rapidly increasing worldwide. This trend is causing enormous economic losses due to the social costs of medical and nursing care and the decline in the labor force of patients and their families, and has thus become a major social issue.

Known representative forms of dementia include Alzheimer's disease, frontotemporal dementia, and Lewy body dementia. In these conditions, specific proteins aggregate and accumulate in the nervous system, presumably leading to the death of nerve cells and the onset of dementia. Specifically, $A\beta$ and tau accumulate in the brain in Alzheimer's disease, tau or TDP-43 in frontotemporal dementia, and $\alpha$-synuclein in Lewy body dementia (Non-Patent Literature 1: Spires-Jones et al., Acta Neuropathol., (2017), 134[2]:187-205).

Anti-dementia drugs that suppress or remove the production of these causative proteins are currently being developed. As candidates for anti-dementia drugs, $A\beta$-producing enzyme inhibitors ($\beta$-secretase and $\gamma$-secretase) (Non-Patent Literature 2: Luo et al., Cell & Biosci., (2022), 12:2), anti-$A\beta$ vaccines (Non-Patent Literature 3: Valiukas et al., Vaccines, (2022), 10[9]:1527) and anti-$A\beta$ antibodies (Non-Patent Literature 4: Song et al., Transl. Neurodegener., (2022), 11:18) have been investigated for Alzheimer's disease; anti-tau vaccines (Non-Patent Literature 5: Medina, Int. J. Mol. Sci., (2018), 19[4]:1160) and anti-tau antibodies (Non-Patent Literature 6: Ji et al., Drugs, (2021), 81[10]:1135-1152) for frontotemporal dementia; and $\alpha$-synuclein inhibitors and anti-$\alpha$-synuclein antibodies (Non-Patent Literature 7: Alzforum website, FBRI LLC, search results for alpha-synuclein targets, https://www.alzforum.org/ therapeutics/search?fda_statuses=&target_types%5B%5D=33416&therapy_types=& conditions=&keywords-entry=&keywords=, according to search conducted in December 2022) for Lewy body dementia.

However, most anti-dementia drug candidates developed thus far have failed to demonstrate the expected efficacy in clinical trials with patients with dementia (Non-Patent Literature 8: Asher et al., Life Sciences, (2022), 306:120861).

Jujube seed (*Ziziphi Spinosae Semen*) is said to have a calming effect on the mind. Jujube seed powder is known as a food ingredient in traditional Eastern medicinal cuisine. Jujube seed extract is known to improve cognitive function (Non-Patent Literature 9: Chinese Traditional and Herbal Drugs, (2001), 32[3]:246-247; Non-Patent Literature 10: Journal of Guangxi Traditional Chinese Medical University, (2002), 5[3]:11-13; and Non-Patent Literature 11: Journal of Xi'an Jiaotong University (Medical Sciences), (2010), 31[6]:673-707). Main ingredients of jujube seed, jujubosides and spinosin, have been reported to have anti-dementia effects (Non-Patent Literature 12: Liu et al., Eur. J. Pharmacol., (2014), 738:206-213; Non-Patent Literature 13: Zhang et al., Theranostics, (2018), 8:4262-4278;Non-Patent Literature 14: Tabassum et al., Sci. Rep., (2019), 9:4512; Non-Patent Literature 15: Ko et al., Biomol. Ther., (2015), 23:156-164; Non-Patent Literature 16: Lee et al., Pharmacol. Biochem. Behav., (2016), 145:9-16; Non-Patent Literature 17: Xu et al., Biomol. Ther., (2019), 27:71-77; Non-Patent Literature 18: Cai et al., Biomol. Ther., (2020), 28:131-136; and Non-Patent Literature 19: Zhang et al., Biomol. Ther., (2020), 28:259-266).

Japanese sweet flag (*Acori Gramineii Tatarinowii Rhizoma*) has commonly been used in traditional Chinese "Kampo" herbal medicine as its root part (hereinafter also referred to as "Japanese sweet flag root") is said to have the ability to improve blood flow and clarify consciousness and be effective for forgetfulness. Main ingredients of Japanese sweet flag root, asarones and eugenol, have been reported to have anti-dementia effects (Non-Patent Literature 20: Geng et al., Biol. Pharm. Bull., (2010), 33:836-843; Non-Patent Literature 21: Liu et al., Yakugaku Zasshi, (2010),130 [5]:737-746; Non-Patent Literature 22: Wei et al., J. Alzheimers Dis., (2013),33:863-880; Non-Patent Literature 23: Yang et al., Cell Mol. Neurobiol., (2016), 36:121-130; Non-Patent Literature 24: Xue et al., Eur. J. Pharmacol., (2014), 741:195-204; Non-Patent Literature 25: Chen et al., Brain Res., (2014), 1552:41-54; and Non-Patent Literature 26: Mao et al., Aging Cell, (2015), 14:784-796). However, the stem of Japanese sweet flag has no clearly stated medicinal properties and is rarely used in traditional Chinese "Kampo" herbal medicine.

In addition, neither jujube seed nor Japanese sweet flag is known to have any effects on promoting nerve cell repair or inducing neurogenesis, removing dementia-causing proteins accumulating in the brain, or treating or preventing neurodegenerative diseases.

## LIST OF CITATIONS

**Non-Patent Literature**

[Non-Patent Literature 1] Spires-Jones et al., Acta Neuropathol., (2017), 134[2]:187-205
[Non-Patent Literature 2] Luo et al., Cell & Biosci., (2022), 12:2
[Non-Patent Literature 3] Valiukas et al., Vaccines, (2022), 10[9]:1527
[Non-Patent Literature 4] Song et al., Transl. Neurodegener., (2022), 11:18
[Non-Patent Literature 5] Medina, Int. J. Mol. Sci., (2018), 19[4]:1160
[Non-Patent Literature 6] Ji et al., Drugs, (2021), 81[10]:1135-1152
[Non-Patent Literature 7] Alzforum website, FBRI LLC, search results for alpha-synuclein targets, https://www.alzforum.org/therapeutics/search?fda_statuses=&target _types%5B%5D=33416&therapy_types=&conditions=&keywords-entry=&keywords =, according to search conducted in December 2022
[Non-Patent Literature 8] Asher et al., Life Sciences, (2022), 306:120861
[Non-Patent Literature 9] Chinese Traditional and Herbal Drugs, (2001), 32[3]:246-247
[Non-Patent Literature 10] Journal of Guangxi Traditional Chinese Medical University, (2002), 5[3]:11-13
[Non-Patent Literature 11] Journal of Xi'an Jiaotong University (Medical Sciences), (2010), 31[6]:673-707
[Non-Patent Literature 12] Liu et al., Eur. J. Pharmacol., (2014), 738:206-213
[Non-Patent Literature 13] Zhang et al., Theranostics, (2018), 8:4262-4278
[Non-Patent Literature 14] Tabassum et al., Sci. Rep., (2019), 9:4512
[Non-Patent Literature 15] Ko et al., Biomol. Ther., (2015), 23:156-164
[Non-Patent Literature 16] Lee et al., Pharmacol. Biochem. Behav., (2016), 145:9-16
[Non-Patent Literature 17] Xu et al., Biomol. Ther., (2019), 27:71-77
[Non-Patent Literature 18] Cai et al., Biomol. Ther., (2020), 28:131-136
[Non-Patent Literature 19] Zhang et al., Biomol. Ther., (2020), 28:259-266
[Non-Patent Literature 20] Geng et al., Biol. Pharm. Bull., (2010), 33:836-843
[Non-Patent Literature 21] Liu et al., Yakugaku Zasshi, (2010),130[5]:737-746
[Non-Patent Literature 22] Wei et al., J. Alzheimers Dis., (2013),33:863-880
[Non-Patent Literature 23] Yang et al., Cell Mol. Neurobiol., (2016), 36:121-130
[Non-Patent Literature 24] Xue et al., Eur. J. Pharmacol., (2014), 741:195-204
[Non-Patent Literature 25] Chen et al., Brain Res., (2014), 1552:41-54
[Non-Patent Literature 26] Mao et al., Aging Cell, (2015), 14:784-796
[Non-Patent Literature 27] Liu et al., Talanta, (2007), 71668-675
[Non-Patent Literature 28] Wang et al., J. Pharm. Anal., (2019), 9[6]:406-413
[Non-Patent Literature 29] Stalder et al., Am. J. Pathol., (1999), 154[6]:1673-1684
[Non-Patent Literature 30] Umeda et al., Am. J. Clin. Pathol., (2013), 183[1]:211-225
[Non-Patent Literature 31] Rota et al., Transl. Neurodegener., (2019), 8:5
[Non-Patent Literature 32] Tomiyama et al., J. Neurosci., (2010), 30[14]:4845-4856
[Non-Patent Literature 33] Umeda et al., Acta Neuropathol. Commun., (2017), 5:59
[Non-Patent Literature 34] Kelley Bromley-Brits et al., J. Vis. Exp., (2011), 53:e2920
[Non-Patent Literature 35] Lippa et al., Arch Neurol., (1999), 561111-1118
[Non-Patent Literature 36] Liu et al., Neuron., (2016), 90(3):521-34
[Non-Patent Literature 37] Hatanaka et al., Biomedicines., (2022), 10(5):1080

## SUMMARY OF INVENTION

### PROBLEM TO BE SOLVED BY THE INVENTION

A problem to be solved in the present invention is to provide a novel means to maintain or improve cerebral functions, to promote nerve cell repair or induce neurogenesis, to remove dementia-causing proteins accumulating in the brain, or to treat or prevent neurodegenerative diseases.

### MEANS TO SOLVE THE PROBLEM

As a result of intensive research, the present inventors have found that jujube seed and the stem portion of Japanese sweet flag, each in specific forms, have various effects such as maintaining or improving cerebral functions, promoting nerve cell repair or inducing neurogenesis, removing dementia-causing proteins accumulating in the brain, and treating or preventing neurodegenerative diseases, and have thereby completed the present invention.

Thus, aspects of the present invention include the following.

[Aspect 1]

An agent for maintaining or improving a cerebral function, the agent comprising an herbal medicine selected from jujube seed (*Ziziphi Spinosae Semen*) and a stem portion of Japanese sweet flag (*Acori Graminei/Tatarinowii Rhizoma*), wherein the jujube seed is in a form selected from a crushed product and an extraction residue of crushed product, and the stem portion of Japanese sweet flag is in a form selected from a crushed product, an extract of crushed product, and an extraction residue of crushed product.

[Aspect 2]

The agent for maintaining or improving a cerebral function according to Aspect 1, wherein the cerebral function is cognitive function.

[Aspect 3]

An agent for promoting nerve cell repair or inducing neurogenesis, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 4]

An agent for removing a dementia-causing protein that accumulates in the brain, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 5]

The agent according to Aspect 4, wherein the dementia-causing protein is one or more selected from amyloid $\beta$ (A$\beta$), tau, $\alpha$-synuclein, TDP-43, FUS/TLS, polyglutamine, protein resulting from RAN (repeat-associated non-ATG) translation, prion, and SOD-1.

[Aspect 6]

An agent for treating or preventing a neurodegenerative disease, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 7]

The agent according to Aspect 6, wherein the neurodegenerative disease is neurodegenerative dementia.

[Aspect 8]

The agent according to Aspect 7, wherein the neurodegenerative dementia is one or more selected from Alzheimer's disease, frontotemporal dementia, and Lewy body dementia.

[Aspect 9]

The agent according to any one of Aspects 1 to 8, wherein the herbal medicine is administered to a subject at an amount of from 0.05mg to 10g per day.

[Aspect 10]

A food product comprising the agent according to any one of Aspects 1 to 8.

[Aspect 11]

A pharmaceutical product comprising the agent according to any one of Aspects 1 to 8.

[Aspect 12]

A method for maintaining or improving a cerebral function in a subject, the method comprising administering to a subject in need thereof an herbal medicine selected from jujube seed (*Ziziphi Spinosae Semen*) and a stem portion of Japanese sweet flag (*Acori Graminei/Tatarinowii Rhizoma*), wherein the jujube seed is in a form selected from a crushed product and an extraction residue of crushed product, and the stem portion of Japanese sweet flag is in a form selected from a crushed product, an extract of crushed product, and an extraction residue of crushed product.

[Aspect 13]

The method according to Aspect 12, wherein the cerebral function is cognitive function.

[Aspect 14]

A method for promoting nerve cell repair or inducing neurogenesis in a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 15]

A method for removing a dementia-causing protein that accumulates in the brain of a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 16]

The method according to Aspect 15, wherein the dementia-causing protein is one or more selected from amyloid β (Aβ), tau, α-synuclein, TDP-43, FUS/TLS, polyglutamine, protein resulting from RAN (repeat-associated non-ATG) translation, prion, and SOD-1.

[Aspect 17]

A method for treating or preventing a neurodegenerative disease in a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

[Aspect 18]

The method according to Aspect 17, wherein the neurodegenerative disease is neurodegenerative dementia.

[Aspect 19]

The method according to Aspect 18, wherein the neurodegenerative dementia is one or more selected from Alzheimer's disease, frontotemporal dementia, and Lewy body dementia.

[Aspect 20]

The method according to any one of Aspects 12 to 19, wherein the herbal medicine is administered to the subject at an amount of from 0.05mg to 10g per day.

[Aspect 21]

Crushed powder of an herbal medicine selected from jujube seed and a stem portion of Japanese sweet flag, wherein the percentage of crushed powder particles with a particle size of 100 μm or less to the total crushed powder particles is 50% or more.

[Aspect 22]

The crushed powder according to Aspect 21, which has a moisture content activity of less than 0.7.

## EFFECT OF THE INVENTION

The present invention provides a novel means to maintain or improve cerebral functions, to promote nerve cell repair or induce neurogenesis, to remove dementia-causing proteins accumulating in the brain, or to treat or prevent neurodegenerative diseases, by using jujube seed and the stem portion of Japanese sweet flag each in specific forms.

## BRIEF EXPLANATION OF FIGURES

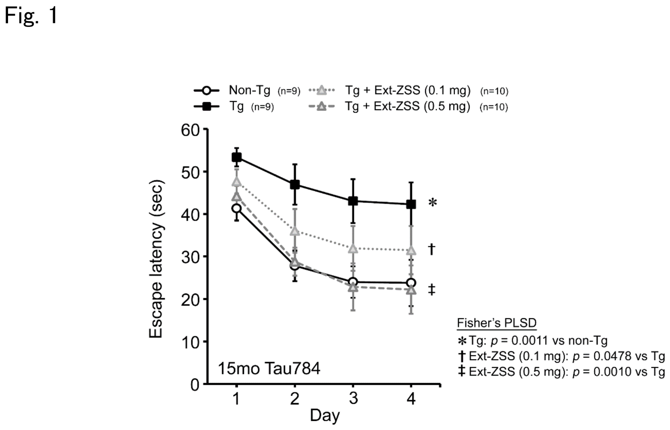

[Figure 1] Figure 1 indicates graphs showing the results of the Morris water maze test of Tau784 mice administered the hot water extract of jujube seed (Ext-ZSS) at 0.1mg per day and 0.5mg per day in Example A1, in comparison with Tau784 mice administered water and non-genetically modified mice.

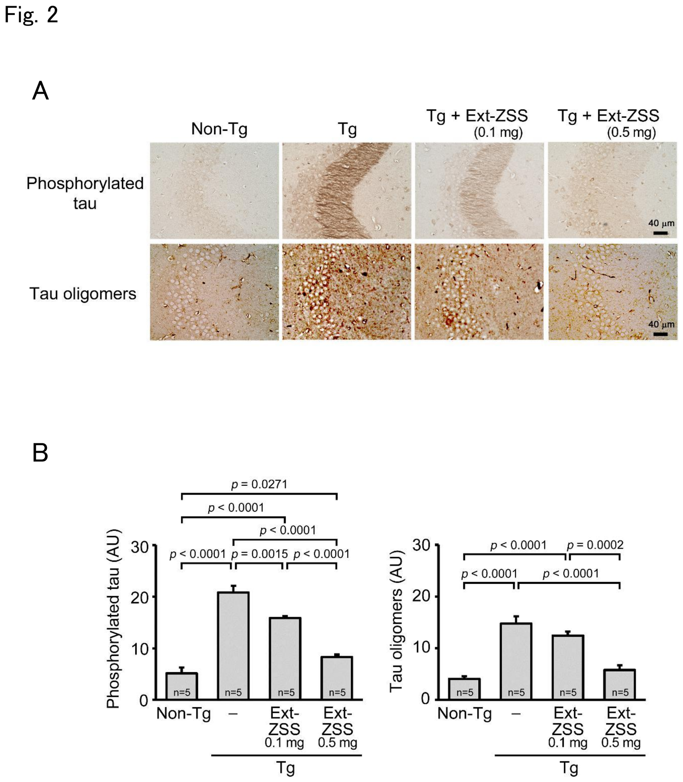

[Figure 2] Figure 2 shows tau pathology in the hippocampal CA2/3 region and cerebral cortex of Tau784 mice administered the hot water extract of jujube seed (Ext-ZSS) at 0.1mg per day and 0.5mg per day in Example A1, in comparison with Tau784 mice administered water and non-genetically modified mice. Specifically, Figure 2A indicates photographs of the staining results for phosphorylated tau (hippocampus) and tau oligomers (cerebral cortex), and Figure 2B indicates graphs showing quantitative values of staining intensity in each photograph.

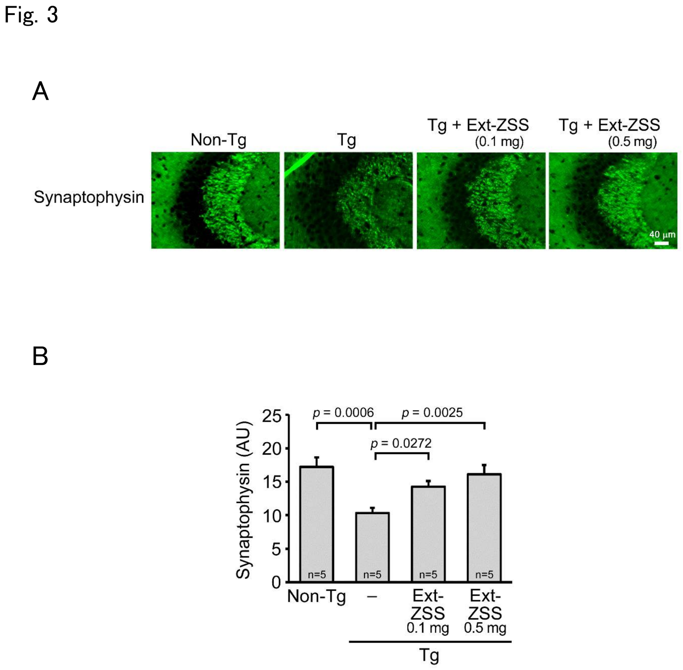

[Figure 3] Figure 3 shows synaptophysin pathology in mossy fibers of the hippocampal CA2/3 region of Tau784 mice administered the hot water extract of jujube seed (Ext-ZSS) at 0.1mg per day and 0.5mg per day in Example A1, in comparison with Tau784 mice administered water and non-genetically modified mice. Specifically, Figure 3A indicates photographs of the staining results of synaptophysin, and Figure 3B indicates graphs showing values of staining intensity in each photograph.

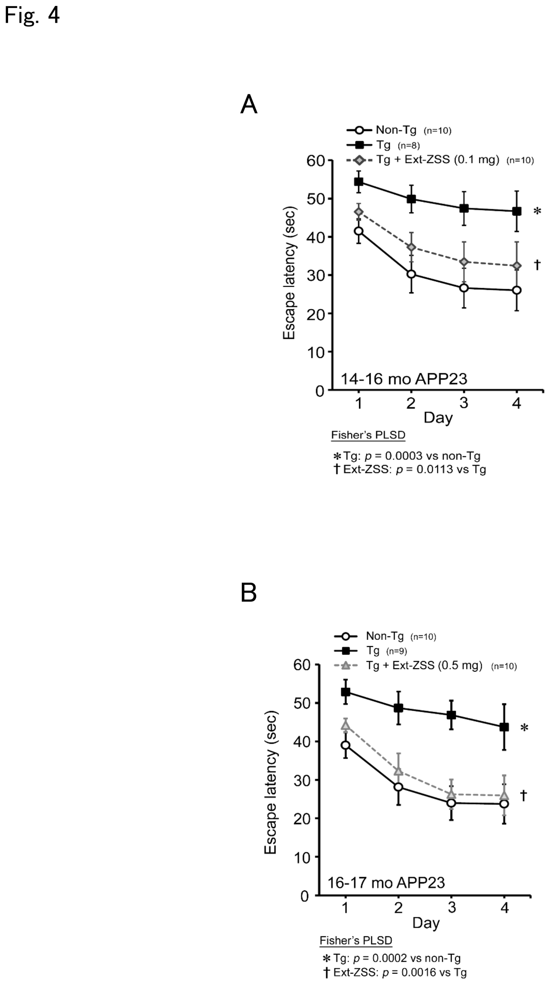

[Figure 4] Figure 4 indicates graphs showing the results of the Morris water maze test of APP23 mice administered the hot water extract of jujube seed (Ext-ZSS) in Example A2, in comparison with APP23 mice administered water and non-genetically modified mice. Specifically, Figure 4A indicates graphs showing the results of administration at 0.1 mg/day, and Figure 4B indicates graphs showing the results of administration at 0.5 mg/day.

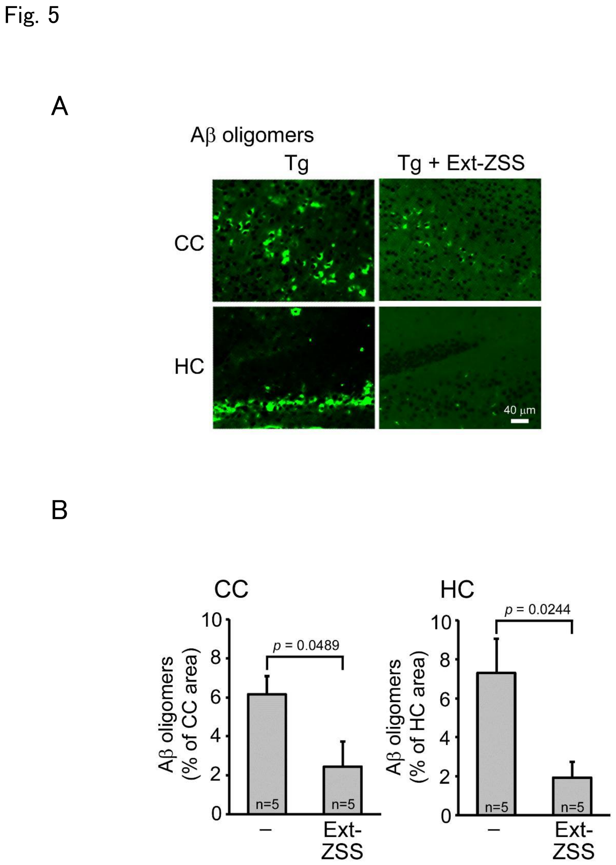

[Figure 5] Figure 5 shows amyloid β oligomer pathology in the cerebral cortex (CC) and hippocampus (HC) of APP23 mice administered the hot water extract of jujube seed (Ext-ZSS) at 0.1mg per day in Example A2, in comparison with APP23 mice administered water. Specifically, Figure 5A indicates photographs of the staining results of amyloid β oligomers, and Figure 5B indicates graphs showing quantitative values of staining intensity in each photograph.

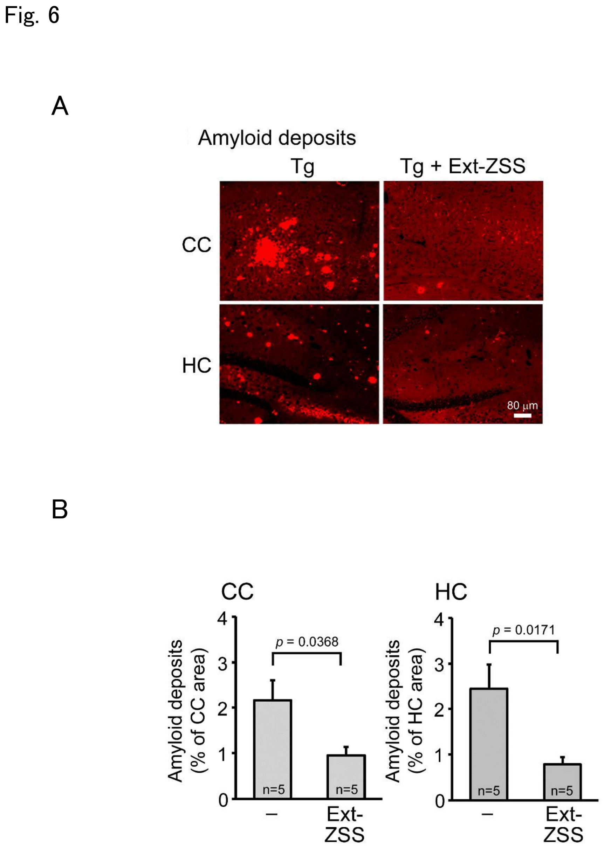

[Figure 6] Figure 6 shows amyloid deposition pathology in the cerebral cortex (CC) and hippocampus (HC) of APP23 mice administered the hot water extract of jujube seed (Ext-ZSS) at 0.1mg per day in Example A2, in comparison with APP23 mice administered water. Specifically, Figure 6A indicates photographs of the staining results of amyloid deposition, and Figure 6B indicates graphs showing quantitative values of staining intensity in each photograph.

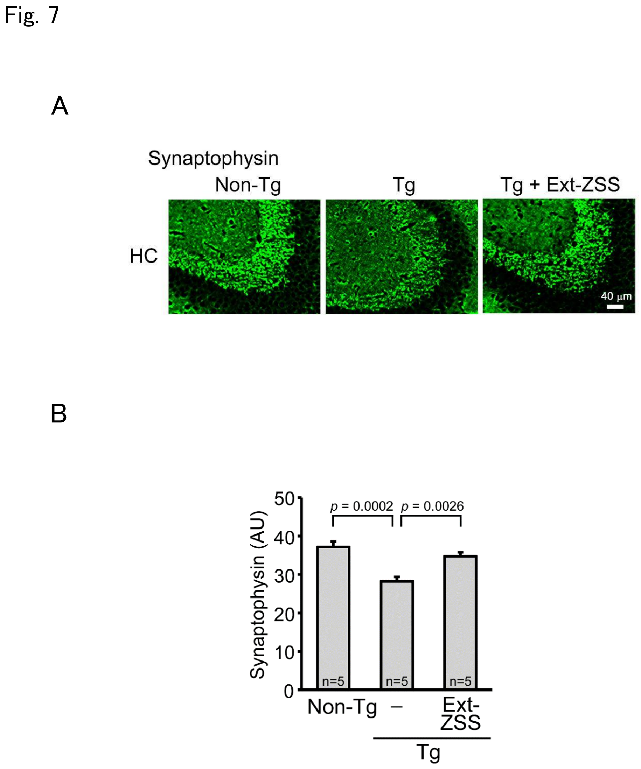

[Figure 7] Figure 7 shows synaptophysin pathology in the hippocampal CA2/3 region of APP23 mice administered the hot water extract of jujube seed (Ext-ZSS) at 0.1mg per day in Example A2, in comparison with APP23 mice administered water and non-genetically modified mice. Figure 7A indicates photographs of the staining results of synaptophysin, and Figure 7B indicates graphs showing quantitative values of staining intensity in each photograph.

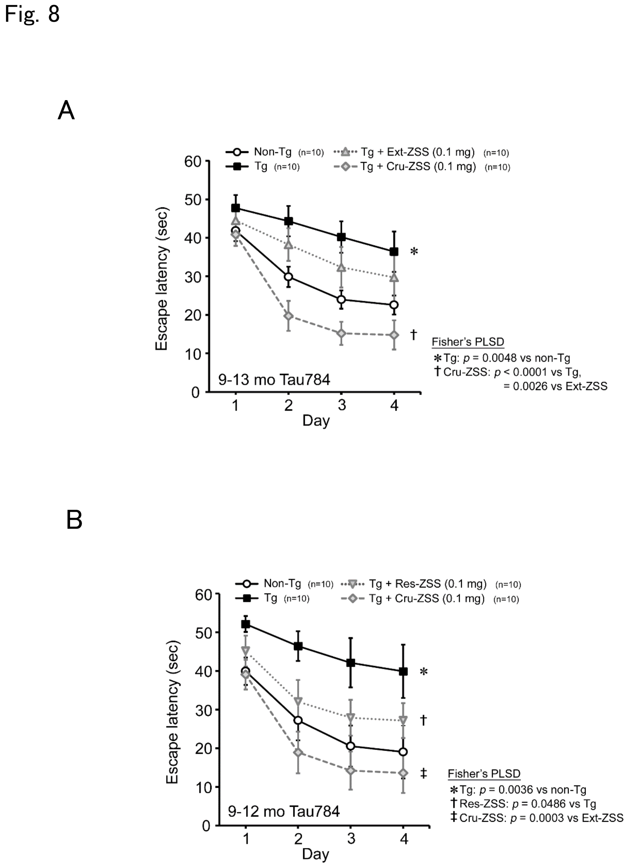

[Figure 8] Figure 8 shows the results of the Morris water maze test Tau784 mice administered the hot water extract (Ext-ZSS), the crushed powder (Cru-ZSS), or the extraction residue (Res-ZSS) of jujube seed at 0.1mg per day in Example A3, in comparison with Tau784 mice administered water and non-genetically modified mice. Specifically, Figure 8A indicates graphs showing the results of administration of hot water extract (Ext-ZSS) and crushed powder (Cru-ZSS), Figure 8B indicates graphs showing the results of administration of extraction residue (Res-ZSS) and crushed powder (Cru-ZSS).

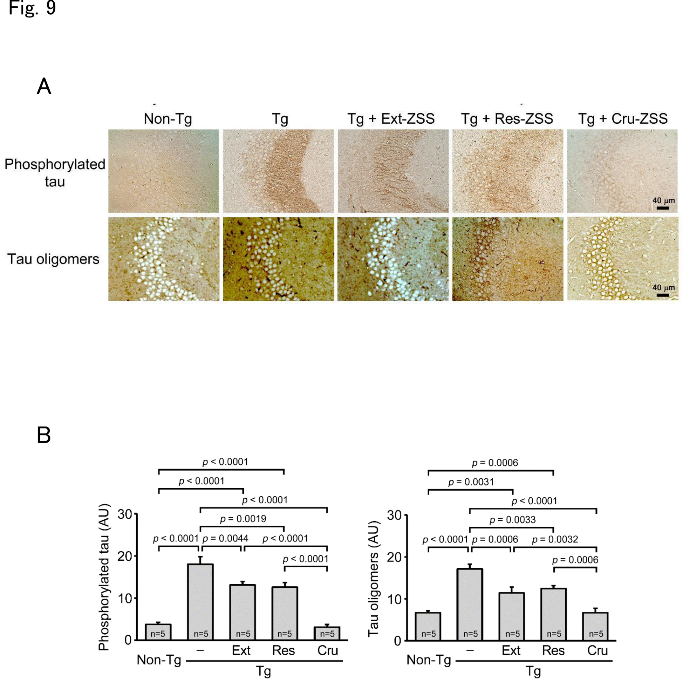

[Figure 9] Figure 9 shows tau pathology in the hippocampal CA2/3 region and cerebral cortex of Tau784 mice administered the hot water extract (Ext-ZSS), the crushed powder (Cru-ZSS), or the extraction residue (Res-ZSS) of jujube seed at 0.1mg per day in Example A3, in comparison with Tau784 mice administered water and non-genetically modified mice. Specifically, Figure 9A indicates photographs of the staining results of phosphorylated tau (hippocampus) and tau oligomer (cerebral cortex), and Figure 9B indicates graphs showing quantitative values of staining intensity in each photograph.

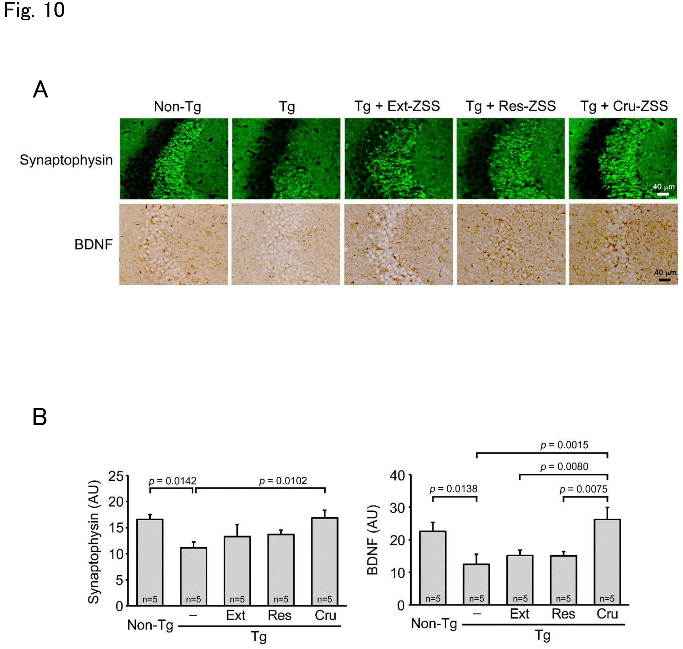

[Figure 10] Figure 10 shows synaptophysin and BDNF pathology in the hippocampal CA2/3 region Tau784 mice administered the hot water extract (Ext-ZSS), the crushed powder (Cru-ZSS), or the extraction residue (Res-ZSS) of jujube seed at 0.1mg per day in Example A3, in comparison with Tau784 mice administered water and non-genetically modified mice. Specifically, Figure 10A indicates photographs of the staining results of synaptophysin and BDNF, Figure 10B indicates graphs showing quantitative values of staining intensity in each photograph.

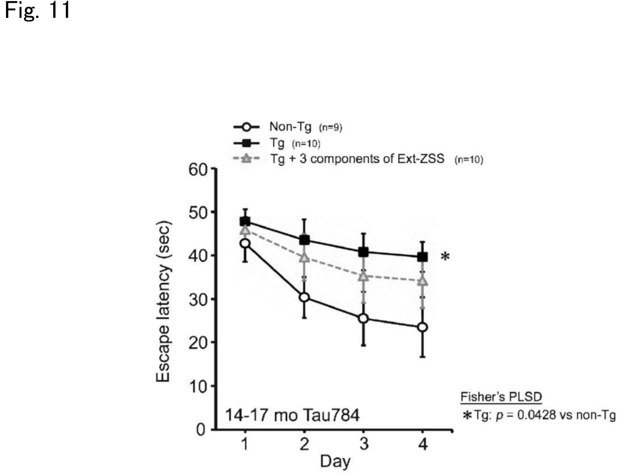

[Figure 11] Figure 11 shows the results of the Morris water maze test of Tau784 mice administered a mixed solution of three main components of jujube seed, i.e., jujuboside A, jujuboside B, and spinosin, in Example A4, in comparison with Tau784 mice administered water and non-genetically modified mice. The daily doses of jujuboside A, jujuboside B, and spinicin in the mixed solution were 0.455 $\mu$g, 0.2 $\mu$g, and 0.7 $\mu$g, respectively, which correspond to the amounts of each component in 0.5 mg of the hot water extract of jujube seed (Ext-ZSS).

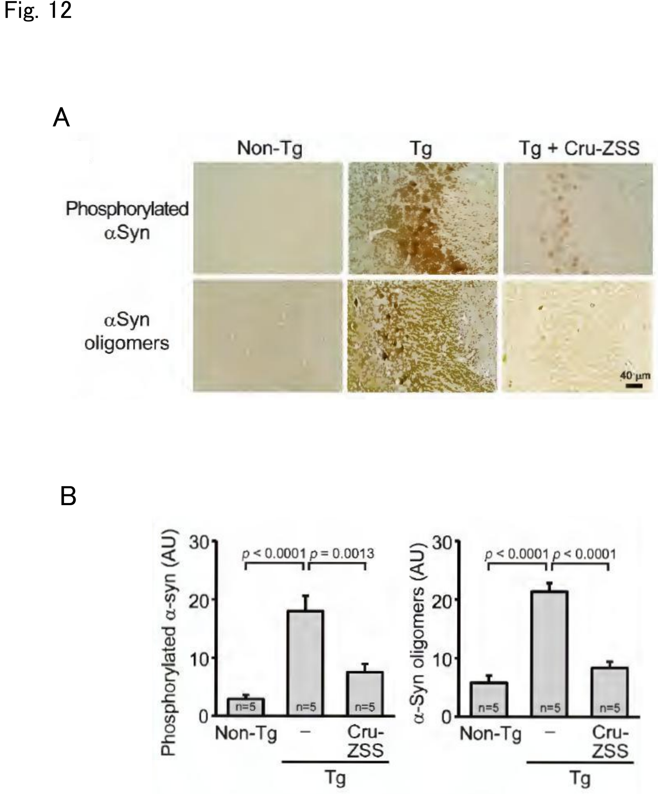

[Figure 12] Figure 12 shows $\alpha$-synuclein pathology in the hippocampal CA2/3 region of Hu$\alpha$-Syn (A53T) mice administered the crushed powder of jujube seed (Cru-ZSS) at 0.1mg per day in Example A5, in comparison with Tau784 mice administered water and non-genetically modified mice. Specifically, Figure 12A indicates photographs of the staining results of phosphorylated $\alpha$-synuclein and $\alpha$-synuclein oligomers, and Figure 12B indicates graphs showing quantitative values of staining intensity in each photograph.

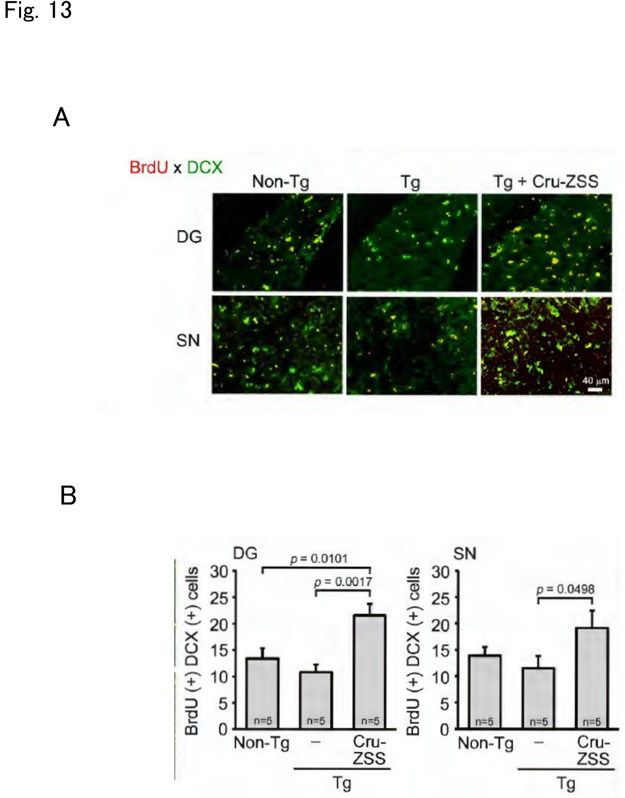

[Figure 13] Figure 13 shows neurogenesis levels in the dentate gyrus (DG) and substantia nigra (SN) of Hu$\alpha$-Syn (A53T) mice administered the crushed powder of jujube seed (Cru-ZSS) at 0.1mg per day in Example A5, in comparison with Hu$\alpha$-Syn (A53T) mice administered water and non-genetically modified mice. Specifically, Figure 13A indicates photographs of immunofluorescence staining results of BrdU (red) and doublecortin (DCX, green), and Figure 13B indicates graphs showing the results of counting double-positive cells (yellow) in each photograph as newborn neurons.

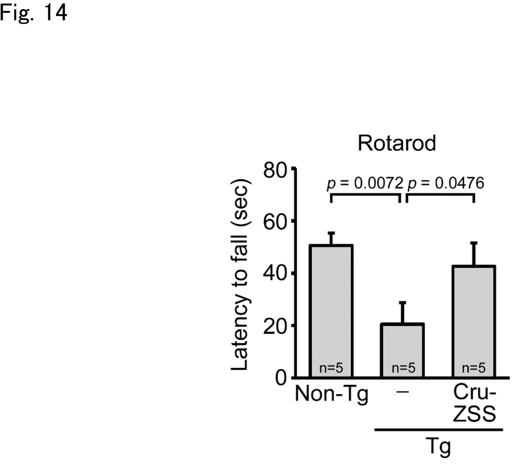

[Figure 14] Figure 14 is a graph showing the result of the rotarod test of Hu$\alpha$-Syn (A53T) mice administered the crushed powder of jujube seed (Cru-ZSS) at 0.1mg per day in Example A6, in comparison with Hu$\alpha$-Syn (A53T) mice administered water and non-genetically modified mice.

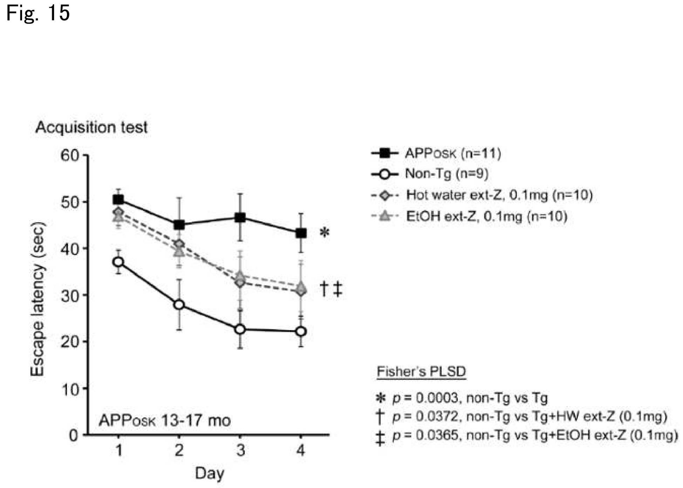

[Figure 15] Figure 15 is a graph showing the results of the Morris water maze test APPOSK mice administered the hot water extract of jujube seed (HOT water ext-Z) or ethanol extract of jujube seed (EtOH ext-Z) at 0.1mg per day in Example A7, in comparison with APPOSK mice administered water and non-genetically modified mice.

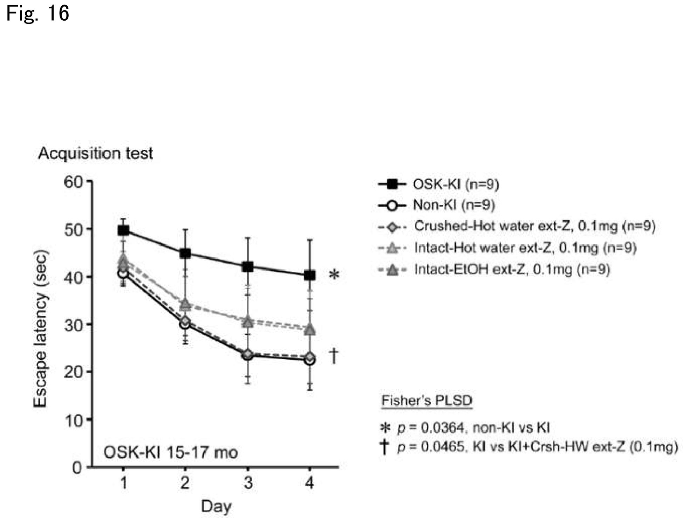

[Figure 16] Figure 16 is a graph showing the results of the Morris water maze test of OSK-KI mice administered the hot water extract of crushed jujube seed (Crushed-HOT water ext-Z), hot water extract of non-crushed jujube seeds (Intact-HOT water ext-Z), or ethanol extract of non-crushed jujube seeds (Intact-EtOH ext-Z) at 0.1mg per day in Comparative Example A8, in comparison with OSK-KI mice administered water and non-genetically modified mice.

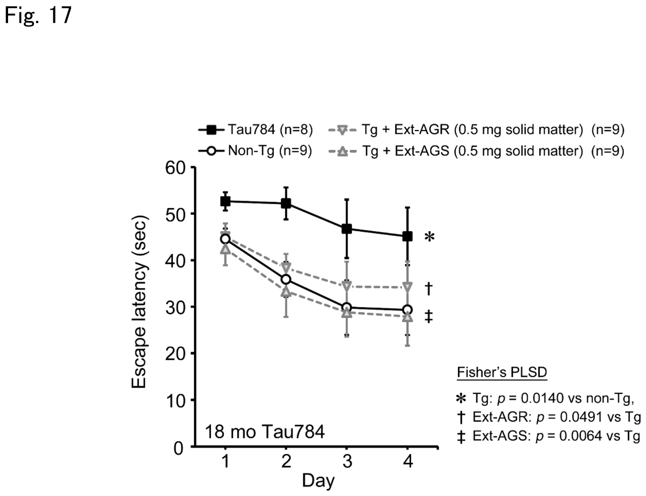

[Figure 17] Figure 17 is a graph showing the results of the Morris water maze test of Tau784 mice administered the hot water extract of Japanese sweet flag stem (Ext-AGS) or hot water extract of Japanese sweet flag root (Ext-AGR) at 0.5mg per day in Example B1, in comparison with Tau784 mice administered water and non-genetically modified mice.

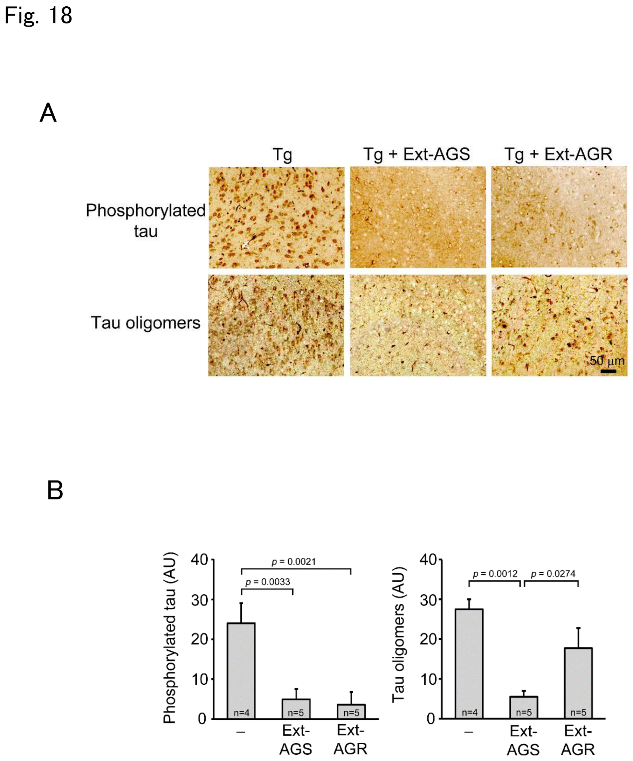

[Figure 18] Figure 18 shows tau pathology in the entorhinal cortex of Tau784 mice administered the hot water extract of Japanese sweet flag stem (Ext-AGS) or hot water extract of Japanese sweet flag root (Ext-AGR) at 0.5mg per day in Example B1, in comparison with Tau784 mice administered water and non-genetically modified mice. Specifically, Figure 18A indicates photographs of the staining results of phosphorylated tau and tau oligomers, and Figure 18B indicates graphs showing quantitative values of staining intensity in each photograph.

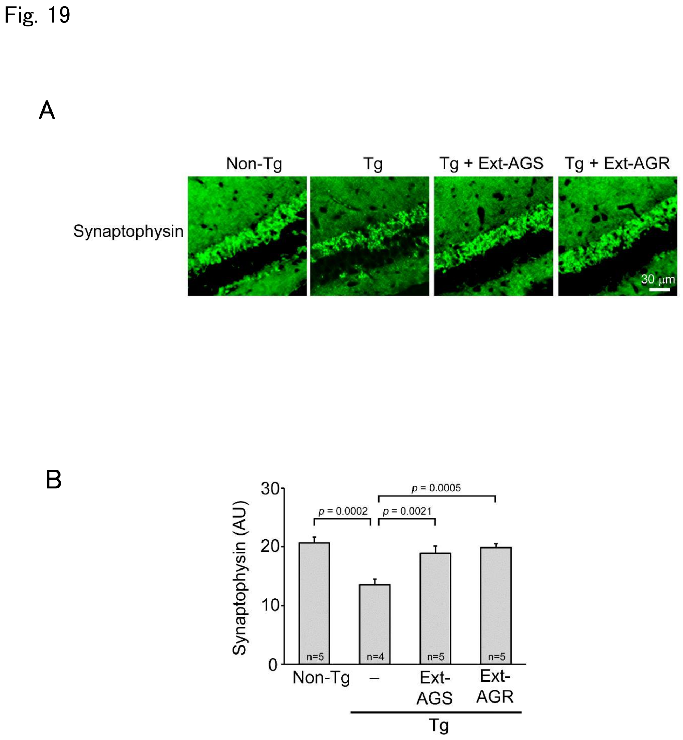

[Figure 19] Figure 19 shows synaptophysin pathology in mossy fibers of the hippocampal CA2/3 region of Tau784 mice administered the hot water extract of Japanese sweet flag stem (Ext-AGS) or hot water extract of Japanese sweet flag root (Ext-AGR) at 0.5mg per day in Example B1, in comparison with Tau784 mice administered water and non-genetically modified mice. Specifically, Figure 19A indicates photographs of the staining results of synaptophysin, and Figure 19B indicates graphs showing quantitative values of staining intensity in each photograph.

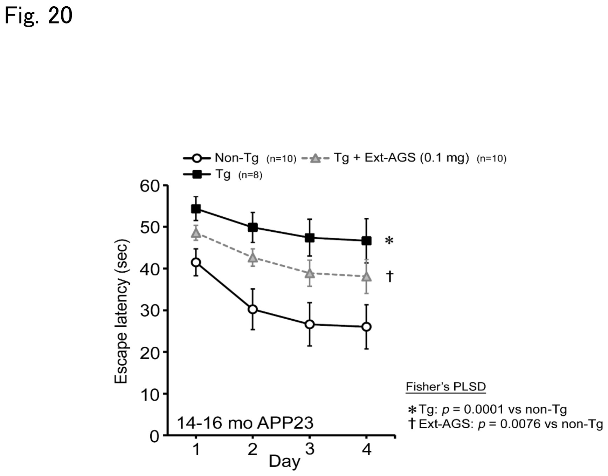

[Figure 20] Figure 20 is a graph showing the results of the Morris water maze test of APP23 mice administered the hot water extract of Japanese sweet flag stem (Ext-AGS) at 0.1mg per day in Example B2, in comparison with APP23 mice administered water and non-genetically modified mice.

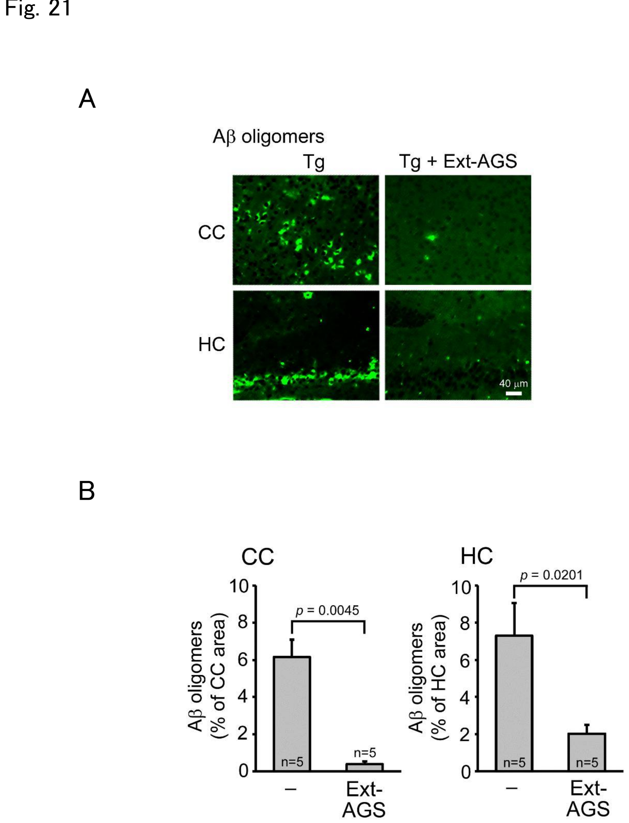

[Figure 21] Figure 21 shows amyloid $\beta$ oligomer pathology in the hippocampus (HC) of APP23 mice administered the hot water extract of Japanese sweet flag stem (Ext-AGS) at 0.1mg per day in Example B2, in comparison with APP23 mice administered water. Specifically, Figure 21A indicates photographs of the staining results of amyloid β oligomers, and Figure 21B indicates graphs showing quantitative values of staining intensity in each photograph.

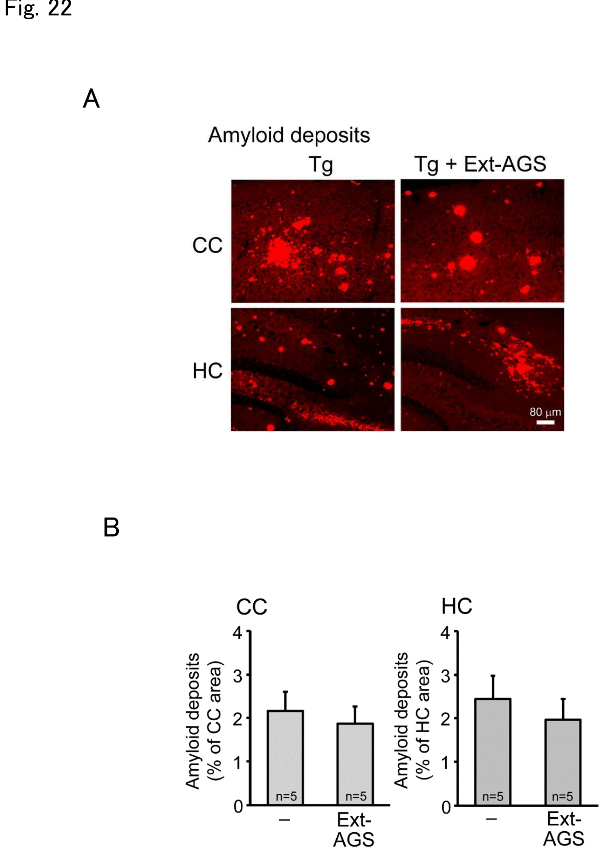

[Figure 22] Figure 22 shows amyloid deposition pathology in the cerebral cortex (CC) and hippocampus (HC) of APP23 mice administered the hot water extract of Japanese sweet flag stem (Ext-AGS) at 0.1mg per day in Example B2, in comparison with APP23 mice administered water. Specifically, Figure 22A indicates photographs of the staining results of amyloid deposition, and Figure 22B indicates graphs showing quantitative values of staining intensity in each photograph.

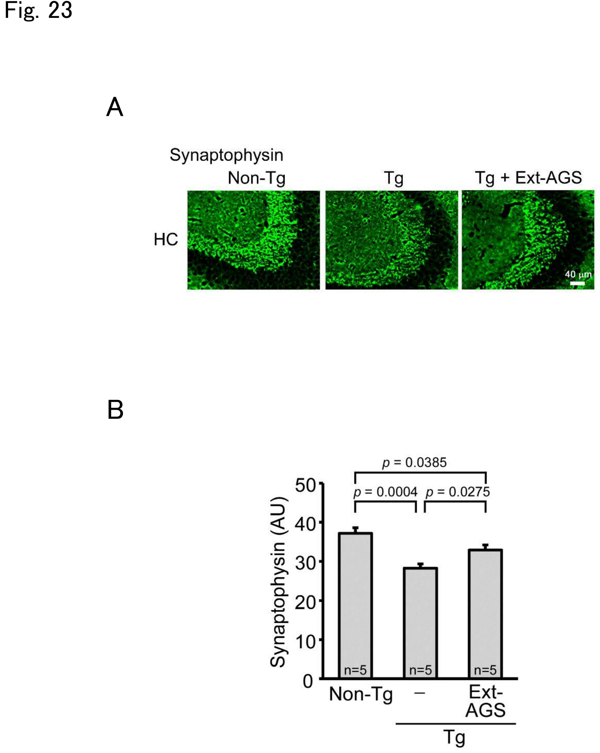

[Figure 23] Figure 23 shows synaptophysin pathology in the hippocampal CA2/3 region of APP23 mice administered the hot water extract of Japanese sweet flag stem (Ext-AGS) at 0.1mg per day in Example B2, in comparison with APP23 mice administered water and non-genetically modified mice. Specifically, Figure 23A indicates photographs of the staining results of synaptophysin, and Figure 23B indicates graphs showing quantitative values of staining intensity in each photograph.

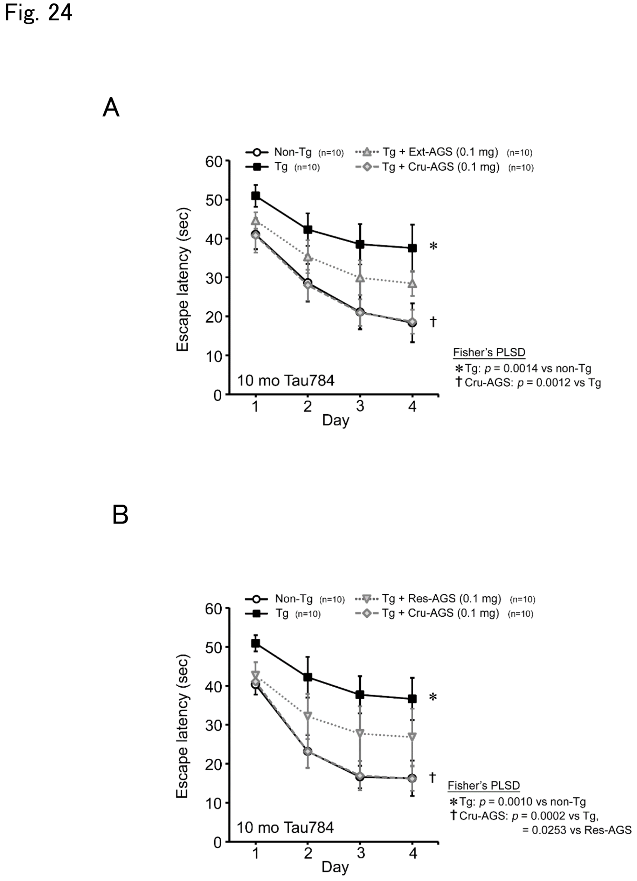

[Figure 24] Figure 24 shows the results of the Morris water maze test of Tau784 mice administered the hot water extract (Ext-AGS), crushed powder (Cru-AGS), or extraction residue (Res-AGS) of Japanese sweet flag stem at 0.1mg per day in Example B3, in comparison with Tau784 mice administered water and non-genetically modified mice. Specifically, Figure 24A is a graph showing the results of administration of hot water extract (Ext-AGS) and crushed powder (Cru-AGS), and Figure 24B is a graph showing the results of administration of extraction residue (Res-AGS) and crushed powder (Cru-AGS).

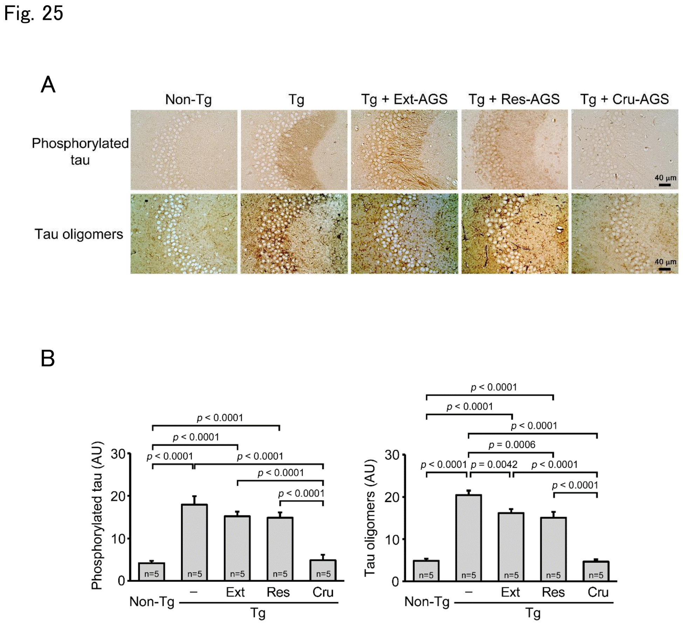

[Figure 25] Figure 25 shows tau pathology in the hippocampal CA2/3 region and cerebral cortex of Tau784 mice administered the hot water extract (Ext-AGS), crushed powder (Cru-AGS), or extraction residue (Res-AGS) of Japanese sweet flag stem at 0.1mg per day in Example B3, in comparison with Tau784 mice administered water and non-genetically modified mice. Specifically, Figure 25A indicates photographs of the staining results of phosphorylated tau (hippocampus) and tau oligomers (cerebral cortex), and Figure 25B indicates graphs showing quantitative values of staining intensity in each photograph.

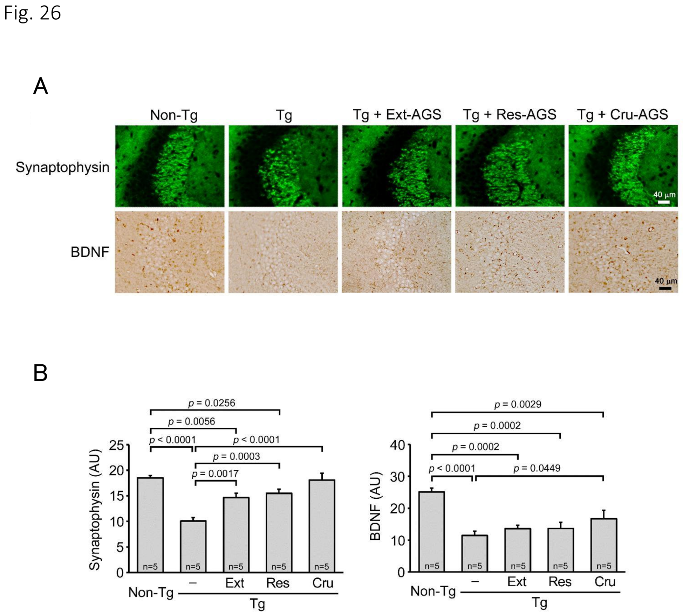

[Figure 26] Figure 26 shows synaptophysin and BDNF pathology in the hippocampal CA2/3 region and cerebral cortex of Tau784 mice administered the hot water extract (Ext-AGS), crushed powder (Cru-AGS), or extraction residue (Res-AGS) of Japanese sweet flag stem at 0.1mg per day in Example B3, in comparison with Tau784 mice administered water and non-genetically modified mice. Specifically, Figure 26A indicates photographs of the staining results of synaptophysin (hippocampus) and BDNF (cerebral cortex), and Figure 26B indicates graphs showing quantitative values of staining intensity in each photograph.

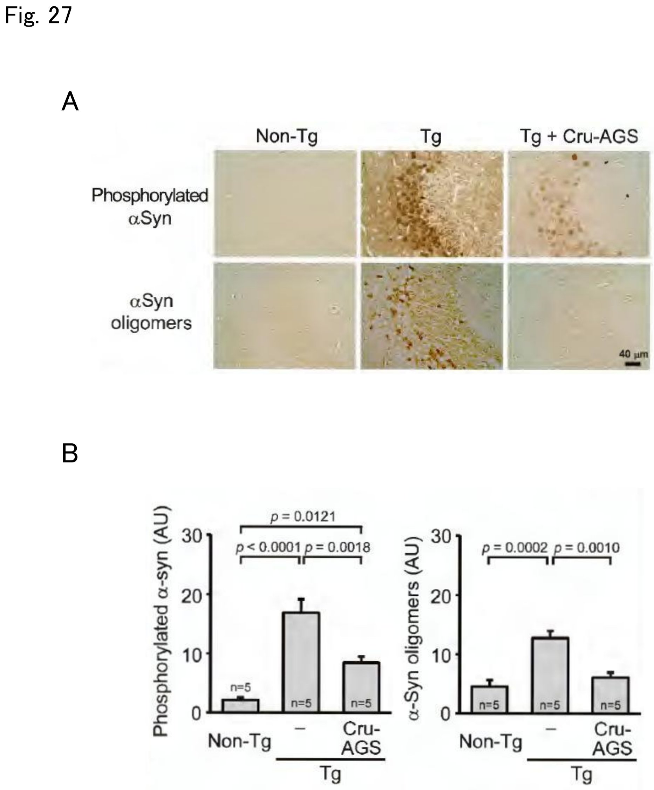

[Figure 27] Figure 27 shows α-synuclein pathology in the hippocampal CA2/3 region of Huα-Syn (A53T) mice administered the crushed powder of Japanese sweet flag stem (Cru-AGS) at 0.1mg per day in Example B4, in comparison with Tau784 mice administered water and non-genetically modified mice. Specifically, Figure 27A indicates photographs of the staining results of phosphorylated α-synuclein and α-synuclein oligomers, and Figure 27B indicates graphs showing quantitative values of staining intensity in each photograph.

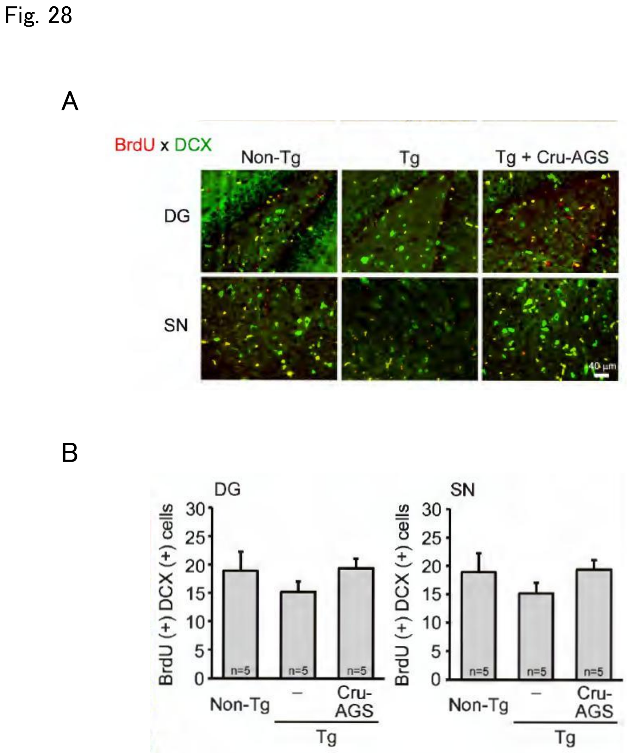

[Figure 28] Figure 28 shows neurogenesis levels in the dentate gyrus (DG) and substantia nigra (SN) of Huα-Syn (A53T) mice administered the crushed powder of Japanese sweet flag stem (Cru-AGS) at 0.1mg per day in Example B4, in comparison with Huα-Syn (A53T) mice administered water and non-genetically modified mice. Specifically, Figure 28A indicates photographs of immunofluorescence staining results of BrdU (red) and doublecortin (DCX, green), and Figure 28B indicates graphs showing the results of counting double-positive cells (yellow) in each photograph as newborn neurons.

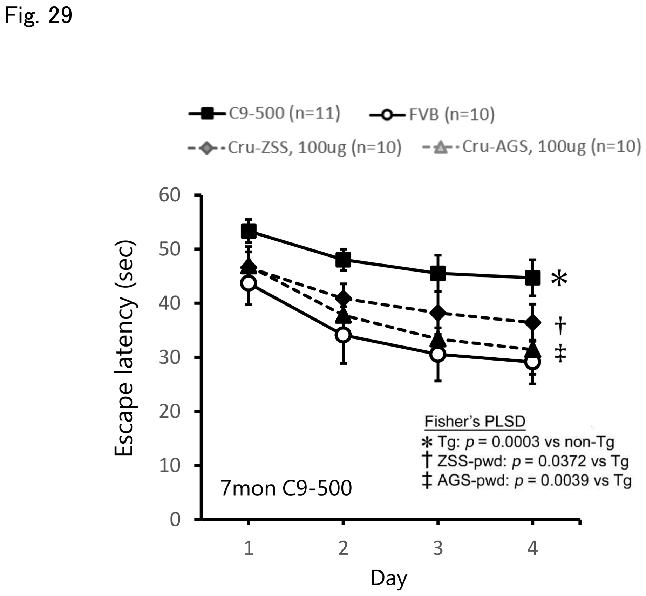

[Figure 29] Figure 29 is a graph showing the results of the Morris water maze test C9-500 mice administered the crushed product of Japanese sweet flag stem (Cru-AGS) or crushed product of jujube seed (Cru-ZSS) at 0.1mg per day in Example C1, in comparison with C9-500 mice administered water and non-genetically modified mice.

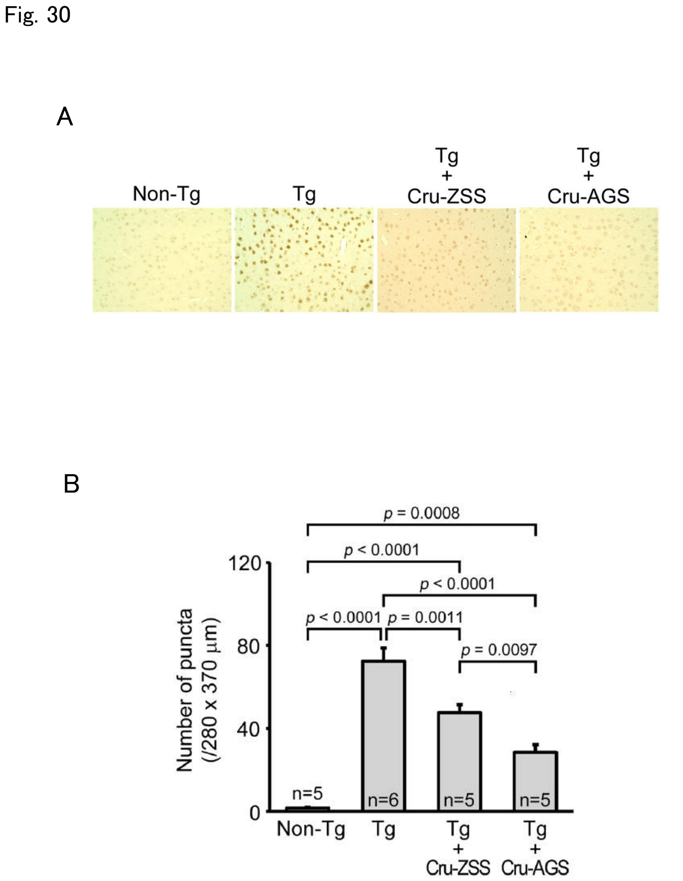

[Figure 30] Figure 30 shows TDP-43 pathology in the hippocampal CA2/3 region of C9-500 mice administered the crushed product of Japanese sweet flag stem (Cru-AGS) or crushed product of jujube seed (Cru-ZSS) at 0.1mg per day, in comparison with C9-500 mice administered water and non-genetically modified mice. Specifically, Figure 30A indicates photographs of the staining results of TDP-43, and Figure 30B indicates graphs showing quantitative values of puncta in each photograph .

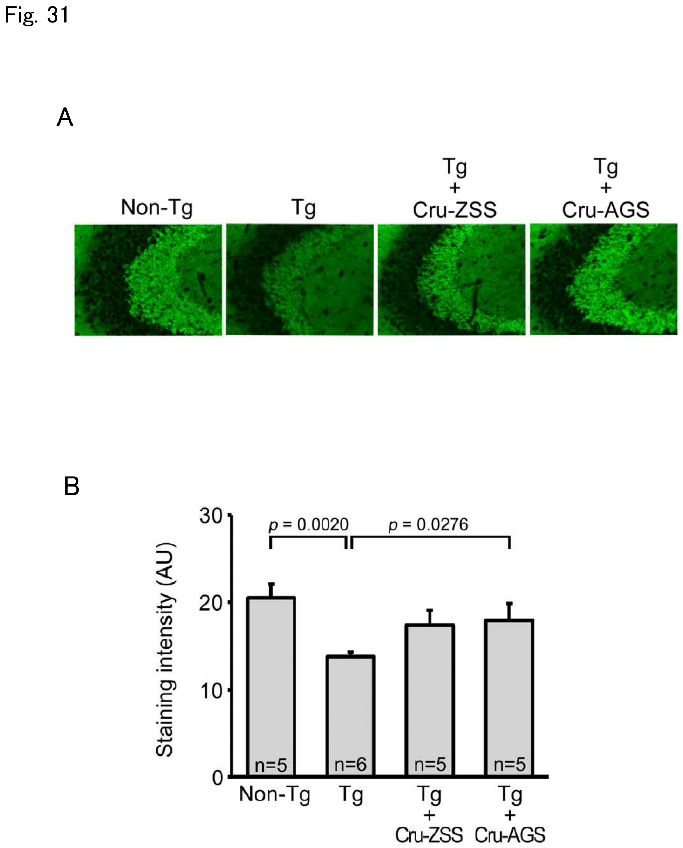

[Figure 31] Figure 31 shows synaptophysin pathology in mossy fibers of the hippocampal CA2/3 region of C9-500 mice administered the crushed product of Japanese sweet flag stem (Cru-AGS) or crushed product of jujube seed (Cru-ZSS) at 0.1mg per day, in comparison with C9-500 mice administered water and non-genetically modified mice. Specifically, Figure 31A indicates photographs of the staining results of synaptophysin, and Figure 31B indicates graphs showing quantitative values of staining intensity in each photograph.

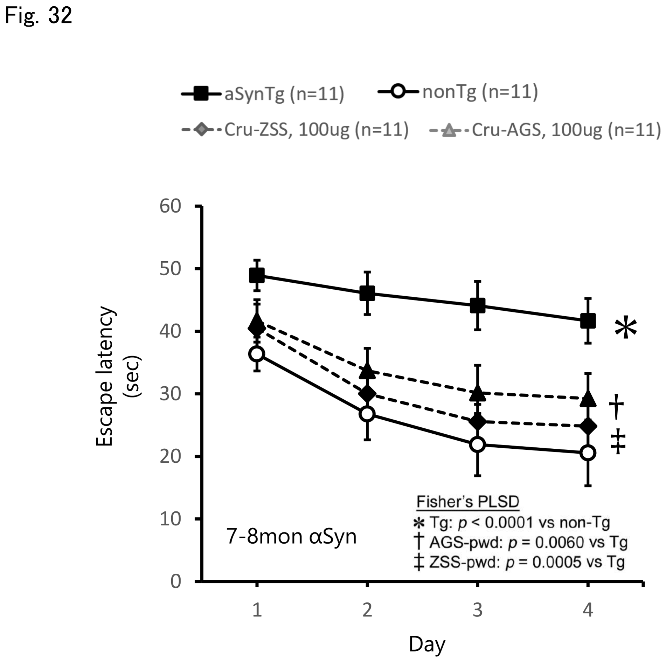

[Figure 32] Figure 32 is a graph showing the results of the Morris water maze test of 6- to 7-month-old Huα-Syn (A53T) mice (average body weight: AGS 28.0g) orally administered the crushed powder of Japanese sweet flag stem (Cru-AGS) and crushed powder of jujube seed (Cru-ZSS) at 0.1mg per day for a month.

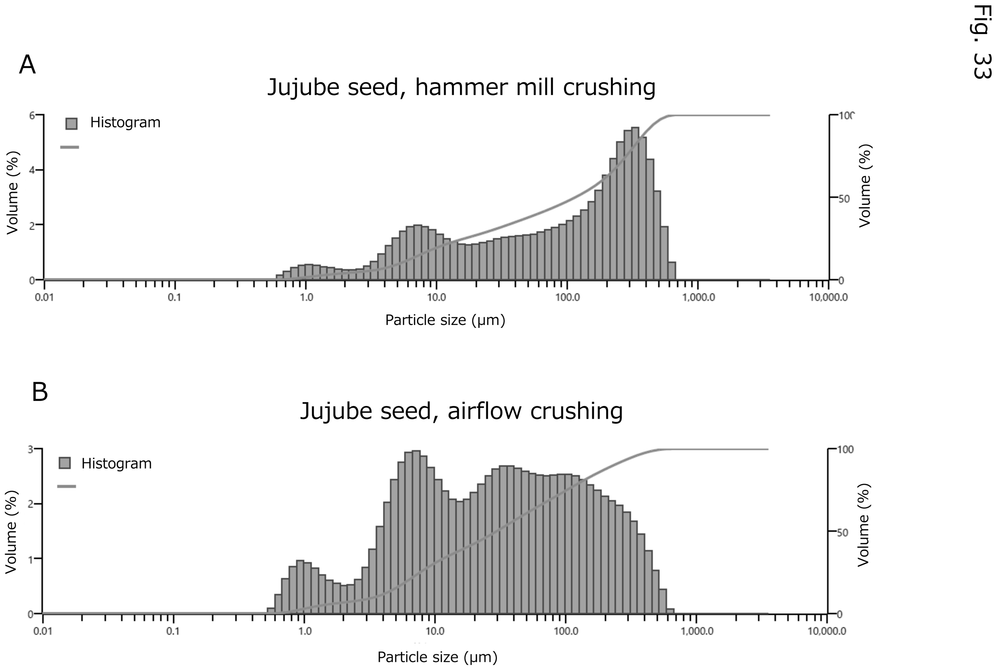

[Figure 33] Figure 33 shows the results of analyzing the particle size distribution of crushed jujube seeds. Specifically, Figure 33A shows the particle size distribution of crushed powder obtained by the hammer mill crushing method, and Figure 33B shows the particle size distribution of crushed powder obtained by the airflow crushing method.

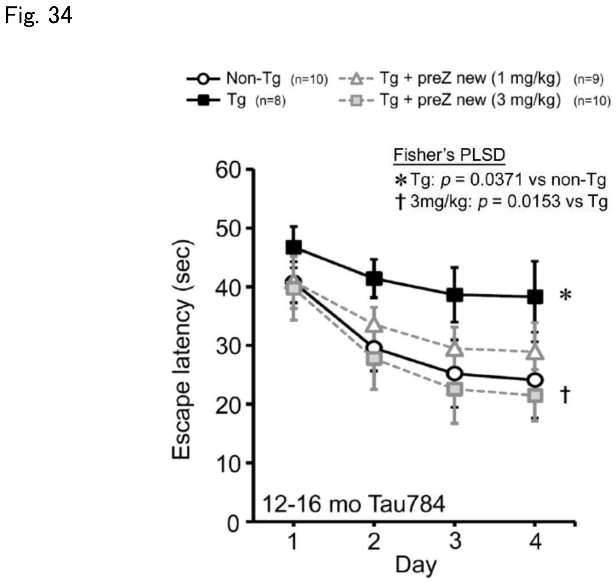

[Figure 34] Figure 34 indicates a graph showing the results of the Morris water maze test of Tau784 mice administered the crushed powder obtained by the airflow crushing method at 1mg/kg per day or 3mg/kg per day in Example D2, in comparison with Tau784 mice administered water and non-genetically modified mice.

## EMBODIMENTS OF THE INVENTIONS

The present invention will now be described in detail with reference to specific embodiments. However, the present invention is not limited to these embodiments and may be implemented in any form unless departing from the scope of the present invention.

[Summary]

As mentioned above, aside from issues related to side effects, the primary reasons why most of the anti-dementia drugs that have been studied to date have failed are the delayed timing of drug administration and targeting the wrong molecular targets. A$\beta$ begins to accumulate in the brain more than 20 years prior to the onset of Alzheimer's disease, and tau begins to accumulate approximately 10 years prior. As A$\beta$ and tau accumulate and neurons begin to die, dementia manifests. In other words, by the time dementia becomes apparent, a significant number of neurons have already died. If the goal is to remove A$\beta$ or tau, it must be done before neurons begin dying. In other words, drugs targeting A$\beta$ or tau should be preventive, not therapeutic. Previously, it was believed that the disease arose when insoluble protein aggregates, such as senile plaques or neurofibrillary tangles (accumulations of aggregated tau), killed neurons. However, recent research suggests that soluble oligomers, which form at an earlier stage, impair neuronal function and lead to the onset of dementia. Therefore, removing these oligomers is necessary to prevent dementia.

From a preventive perspective, it would be preferable for a single drug to act on various causative protein oligomers rather than being specific to A$\beta$, tau, or $\alpha$-synuclein. Additionally, damaged nerve cells must be repaired and brain function restored. Furthermore, since preventing dementia is a long-term process, preventive medications should be safe and affordable. Ideally, they should also be self-administered non-invasively, without the need for medical assistance. Given the numerous requirements for dementia preventive medications, it is challenging to achieve them with a single-component pharmaceutical. Furthermore, if all middle-aged and older adults were to take these medications long-term to prevent dementia, it could eventually lead to the collapse of the healthcare economy.

To address these challenges, the present inventors have focused on herbal medicines, which are used in traditional Chinese "Kampo" herbal medicine with a long history. If effective herbal medicines for improving cognitive function existed, middle-aged and elderly people could buy them without consulting a doctor and add them to their diet for consumption, whereby they could work to prevent dementia in their daily lives. Based on this idea, the present inventors have conducted intensive research and found that jujube seed and the stem portion of Japanese sweet flag (Japanese sweet flag stem), each in specific forms, have various effects such as maintaining or improving cerebral functions, promoting nerve cell repair or inducing neurogenesis, removing dementia-causing proteins accumulating in the brain, and treating or preventing neurodegenerative diseases. The present invention is based on these findings.

Thus, an aspect of the present invention provides an agent for maintaining or improving a cerebral function, an agent for promoting nerve cell repair or inducing neurogenesis, an agent for removing a dementia-causing protein accumulating in the brain, and an agent for treating or preventing a neurodegenerative disease, each containing an herbal medicine selected from jujube seed and a stem portion of Japanese sweet flag (which may also be referred to collectively as an "agent of the present invention").

Another aspect of the present invention provides a food product containing an agent of the present invention (which may also be referred to as a "food product of the present invention").

Still another aspect of the present invention provides a pharmaceutical product containing an agent of the present invention (which may also be referred to as a "pharmaceutical product of the present invention").

Still another aspect of the present invention provides a method for maintaining or improving a cerebral function, a method for promoting nerve cell repair or inducing neurogenesis, a method for removing a dementia-causing protein accumulating in the brain, and a method for treating or preventing a neurodegenerative disease, each including administering to a subject an herbal medicine selected from jujube seed and a stem portion of Japanese sweet flag (which may also be referred to collectively as a "method of the present invention").

[Jujube seed]

According to an embodiment, the agent of the present invention may contain jujube seed (*Ziziphi Spinosae*

*Semen,* "酸棗仁") as an herbal medicine. In traditional Chinese "Kampo" herbal medicine Jujube seed is said to have a "calming effect" and is used in combination with ingredients such as polygala root (*polygalae radix*) and longan aril (*longan arillus*) to treat insomnia and forgetfulness. According to the PMDA notification (explained later), jujube seed is not classified as a drug and can be used as a food ingredient.

The present inventors prepared powder of dried and crushed jujube seed (Cru-ZSS), an extract of the dried and crushed jujube seed powder with hot water (Ext-ZSS), and the extract residue remaining after the extraction (Res-ZSS), administered them orally to model mice of tau pathology-positive frontotemporal dementia (Tau784 mice: Examples A1, A3, and A4), model mice of TDP-43 pathology-positive frontotemporal dementia (C9-500 mice: Example C1), and model mice of Aβ pathology-positive Alzheimer's disease (APP23 mice: Example A2) for one month, and evaluated their effects in improving their cognitive functions and brain pathologies. The present inventors also administered the dried and crushed jujube seed powder (Cru-ZSS) orally for one month to model mice of Lewy body dementia showing α-synuclein pathology (Huα-Syn (A53T) mice: Example A6) for one month and evaluated its effects in improving their brain pathology and promoting neurogenesis. The present inventors further evaluated the neuroprotective effect of jujube seed based on its effects in inducing brain-derived neurotrophic factor (BDNF) expression and inducing neurogenesis. As a result, it was found that jujube seed had various effects on the model mice, such as improvement in the cognitive functions, removal of Aβ, tau, and α-synuclein oligomers from the brain, synapse repair, increased expression of brain-derived neurotrophic factor (BDNF), and increased neurogenesis, not only in the form of hot water extract (Ext-ZSS), which form is commonly used in the traditional "Kampo" herbal medicine, but also in the forms of extraction residue (Res-ZSS), and as mere crushed powder (Cru-ZSS). It is also noteworthy that these effects were strongest in crushed jujube seed powder and that mice given the powder performed better on cognitive tests than wild-type mice of the same age.

Main components of jujube seed include spinosin, jujuboside A, and jujuboside B. These components are already known to have the effects of removing Aβ, inducing BDNF expression, and promoting neurogenesis (Non-Patent Literature 12: Liu et al., Eur. J. Pharmacol., (2014), 738:206-213; Non-Patent Literature 13: Zhang et al., Theranostics, (2018), 8:4262-4278; Non-Patent Literature 14: Tabassum et al., Sci. Rep., (2019), 9:4512; Non-Patent Literature 15: Ko et al., Biomol. Ther., (2015), 23:156-164; Non-Patent Literature 16: Lee et al., Pharmacol. Biochem. Behav., (2016), 145:9-16; Non-Patent Literature 17: Xu et al., Biomol. Ther., (2019), 27:71-77; Non-Patent Literature 18: Cai et al., Biomol. Ther., (2020), 28:131-136; and Non-Patent Literature 19: Zhang et al., Biomol. Ther., (2020), 28:259-266, all mentioned above). The present inventors compared the contents of these components in the dried and crushed jujube seed powder (Cru-ZSS) and the hot water extract of dried jujube seed powder (Ext-ZSS), and found that the extract (Ext-ZSS) contained higher amounts of these components (Example A5), even though the powder (Cru-ZSS) exhibited stronger cognitive function-improving effects (Example A4). The present inventors also administered a mixture of spinosin, jujuboside A, and jujuboside B in the same amounts as contained in the extract (Ext-ZSS) to mice, and found that the cognitive function improvement effect was weaker than that observed when the extract (Ext-ZSS) was administered (Example A5). These results suggest that jujube seed contains additional components that are effective in improving cognitive function, in addition to spinosin, jujuboside A, and jujuboside B, and that these additional active components are most abundant in crushed powder.

[Japanese sweet flag stem]

According to an embodiment, the agent of the present invention may contain a stem portion of Japanese sweet flag (*Acori Graminei Rhizoma,* "石菖蒲") (hereinafter also referred to as "Japanese sweet flag stem") as an herbal medicine. The root of Japanese sweet flag (hereinafter also referred to as "Japanese sweet flag root") is said to have the ability to improve blood flow and clarify consciousness and be effective for forgetfulness and has been commonly used in traditional Chinese "Kampo" herbal medicine. According to a notification from the Director-General of the Pharmaceutical Affairs Bureau, Ministry of Health and Welfare: "Guidance and Regulation on Unapproved and Unauthorized Drugs" (revised on April 18, 2018), Annex: "Standards for the Scope of Drugs," Appendix 3: "List of Substances (Raw Materials) Not Regarded as Drugs Unless Medicinal Efficacy or Effects Are Claimed" (also referred to simply as "the PMDA notification"), Japanese sweet flag root is classified as a drug. However, Japanese sweet flag stem has not been clearly proven for any effects and is rarely used in traditional Chinese "Kampo" herbal medicine. According to the PMDA notification, Japanese sweet flag stem is not classified as a drug and can be used as a food ingredient.

The present inventors prepared powder of dried and crushed Japanese sweet flag stem (Cru-AGS), an extract of the dried and crushed Japanese sweet flag stem powder with hot water (Ext-AGS), and the extract residue remaining after the extraction (Res-AGS), administered them orally to model mice of tau pathology-positive frontotemporal dementia (Tau784 mice: Examples B1 and B3), model mice of Aβ pathology-positive Alzheimer's disease (APP23 mice: Example B2), and model mice of TDP-43 pathology-positive frontotemporal dementia (C9-500 mice: Example C1) for one month, and evaluated their effects in improving their cognitive functions and brain pathologies. The present inventors also administered the dried and crushed Japanese sweet flag stem powder (Cru-AGS) orally for one month to model mice of Lewy body dementia showing α-synuclein pathology (Huα-Syn (A53T) mice: Example B4) for one month and evaluated its effects in improving their brain pathology and promoting neurogenesis. The present inventors further evaluated the neuroprotective effect of Japanese sweet flag stem based on its effects in inducing brain-derived neurotrophic factor (BDNF) expression and inducing neurogenesis. As a result, it was found that Japanese sweet flag stem had various effects on the model mice, such as improvement in the cognitive functions, removal of Aβ, tau, and α-synuclein oligomers from the brain, synapse repair, increased expression of brain-derived neurotrophic factor (BDNF), and increased neurogenesis, not only in the form of hot water extract (Ext-AGS), which form is commonly used in the traditional "Kampo" herbal medicine, but also in the forms of extraction residue (Res-AGS), and as mere crushed powder (Cru-AGS). It is also noteworthy that these effects were strongest in crushed Japanese sweet flag stem powder (Res-AGS) and that mice given the powder performed better on cognitive tests than wild-type mice of the same age.

Main components of Japanese sweet flag root used in traditional Chinese "Kampo" herbal medicine include α-asarone, β-asarone, and eugenol. These components are already known to have the effects of removing dementia-causing proteins such as amyloid β, tau, and α-synuclein and inducing expression of factors such as nerve growth factor (NGF), brain-derived neurotrophic factor (BDNF), glia-derived neurotrophic factor (GDNF) (Non-Patent Literature 20: Geng et al., Biol. Pharm. Bull., (2010), 33:836-843; Non-Patent Literature 21: Liu et al., Yakugaku Zasshi, (2010), 130:737-746; Non-Patent Literature 22: Wei et al., J. Alzheimers Dis., (2013), 33:863-880; Non-Patent Literature 23: Yang et al., Cell Mol. Neurobiol., (2016), 36:121-130; Non-Patent Literature 24: Xue et al., Eur. J. Pharmacol., (2014), 741:195-204; Non-Patent Literature 25: Chen et al., Brain Res., (2014), 1552:41-54; and Non-Patent Literature 26: Mao et al., Aging Cell, (2015), 14:784-796, all mentioned above). However, it was first revealed by the present inventors that Japanese sweet flag stem, which contains relatively small amounts of these components and is not commonly used, have a strong cognitive function-improving effect, in contrast to Japanese sweet flag root, which contains large amounts of these components.

[Form of herbal medicines]

According to an embodiment, jujube seed can be used in any form. Examples of such forms include, but are not limited to, a crushed product of jujube seed, an extract of crushed product, and an extract residue of crushed product. Among these, when used in the agent of the present invention for maintaining or improving cerebral function, jujube seed is usually used in the form of crushed product or extract residue of crushed product, although it may preferably be used in the form of crushed product. On the other hand, when used in the agents of the present invention for promoting nerve cell repair or inducing neurogenesis, removing dementia-causing proteins accumulating in the brain, and treating or preventing neurodegenerative diseases, jujube seed may be in the form of crushed product, extract of crushed product, or extract residue of crushed product, although it may preferably be used in the form of crushed product.

According to an embodiment, Japanese sweet flag stem can be used in any form. Examples of such forms include, but are not limited to, a crushed product of Japanese sweet flag stem, an extract of crushed product, and an extract residue of crushed product. Among these, Japanese sweet flag stem may preferably be used in the form of crushed product.

In traditional Chinese "Kampo" herbal medicine, it is common to extract dried herbal medicines with hot water (i.e., decoct them) and discard the remaining residue. However, according to the findings of the present inventors, herbal medicines selected from jujube seed and Japanese sweet flag stem exhibit the strongest cognitive function improvement effects when used in the form of crushed products, such as powder after being dried. This is a surprising finding that cannot be predicted from the conventional knowledge of traditional Chinese "Kampo" herbal medicine.

Specifically, when an herbal medicine selected from jujube seed and Japanese sweet flag stem is crushed into powder or other form of crushed products, there are no particular restrictions on the processing conditions, examples of which are as follows. First, the herbal medicine is dried. There are no restrictions on the drying conditions, and any known conditions may be used, examples including natural drying and heat drying. The temperature during drying may range from 0 to 100°C. Roasting may also be performed. Next, the dried or roasted herbal medicine is crushed. The methods of crushing are not limited, and any known methods may be used, examples including manual crushing, wet crushing, dry crushing, and other methods using crushing machines. Examples of dry crushing methods include hammer mill crushing and airflow crushing. In the airflow crushing method, a rotating rotor and blades generate a swirling airflow within the crushing chamber, and the fed material is repeatedly collided with each other by the swirling airflow, thereby being ground. The finely crushed material is then moved to a recovery chamber called a classification chamber, while inadequately crushed material is returned to the crushing chamber for further crushing. The crushed herbal medicine can be used as is, or it can be sieved to obtain a powder with a particle size controlled to a predetermined value or less. For example, a sieve with a mesh size of 0.02 mm to 20 mm can be used. More specifically, jujube seed powder can be adjusted to a particle size by passing it through a sieve with a mesh size of 2 to 3 mm and then through a sieve with a mesh size of 500 μm, and Japanese sweet flag stem powder can be adjusted to a particle size by passing it through a sieve with a mesh size of 2 to 3 mm and then through a sieve with a mesh size of 500 μm.

From the viewpoint of usability, it may be preferable to adjust the particle size of the crushed herbal medicine powder so that the particles having a particle size of less than a predetermined particle size. Specifically, it may be desirable that the particles having a particle size of 100 μm or less account for 50% or more, preferably 75% or more, of the total crushed product. In addition, from the viewpoint of preservability, it is desirable that the final crushed powder may preferably have a water activity of less than 0.7, more preferably less than 0.64, and even more preferably less than 0.6. It should be noted that such a crushed herbal medicine product in which the particles having a predetermined particle size or less account for a predetermined ratio or more is also included in the subject of the present invention.

When an herbal medicine selected from jujube seed and Japanese sweet flag stem is made in the form of an extract or an extract residue of crushed product, there are no particular restrictions on the processing conditions, but extraction may be performed by adding an extraction solvent to the dried crushed product of the herbal medicine. Specifically, extraction can be performed by adding, e.g., 1 to 100 parts by mass of extraction solvent to 1 part by mass of dried and crushed herbal medicine and holding it for, e.g., 0.1 to 100 hours. There are no particular restrictions on the extraction solvent, and various well-known solvents can be used, examples including water and hydrophilic organic solvents such as ethanol. Extraction using an extraction solvent may be carried out either at ordinary temperature or elevated temperature. For example, when it is carried out with water under heating, the crushed product can be heated at 30 to 100°C with the addition of hot water. If necessary, stirring, ultrasonic treatment, heating reflux treatment, etc., can be used in combination to increase the extraction efficiency. After extraction is complete, the liquid and solid components are separated manually or by filtration. The separated liquid component can be used as the extract of the crushed product, and the solid component can be used as the extract residue of the crushed product. If necessary, the solvent can be evaporated and concentrated before use.

[Effects of the agents]

According to an embodiment, the agent of the present invention is for maintaining or improving a cerebral function. The statement "maintaining or improving a cerebral function" herein refers to, although is not limited to, maintaining or improving cognitive function and/or motor function.

Maintaining or improving "cognitive function" herein may refer to, although is not limited to, preventing the occurrence of, or improving, abnormalities such as mild forgetfulness, memory impairment seen in dementia, disorientation, impaired judgment and understanding, executive dysfunction, apraxia, agnosia, and aphasia.

Maintaining or improving "motor function" herein may refer to, although is not limited to, preventing the occurrence of, or improving, abnormalities such as tremor, muscle rigidity, postural reflex disorders, akinesia/bradykinesia, and other Parkinsonian symptoms observed in Parkinson's disease; involuntary movements observed in Huntington's disease; coordination disorders observed in spinocerebellar degeneration; and muscle weakness observed in amyotrophic lateral sclerosis.

The effects of maintaining or improving a cerebral function by the agent of the present invention can be evaluated, e.g., as mentioned in the Examples described below, by administering the agent to model animals that develop brain pathology, and then subjecting the animals to a cognitive function test such as the Morris water maze test and/or a motor function test such as the rotarod test, or by staining and observing brain sections of the animals.

According to an embodiment, the agent of the present invention is for promoting nerve cell repair or inducing neurogenesis. The statement "promoting nerve cell repair" herein may refer to, although is not limited to, promoting the recovery of the number and function of synapses that have decreased, promoting the recovery of the function of nerve cells that have decreased, promoting the maintenance and recovery of the function of synapses and nerve cells, and promoting the expression of brain-derived neurotrophic factor (BDNF) and nerve growth factor (NGF), which protect nerve cells from various stresses. The statement "inducing neurogenesis" herein may refer to, although is not limited to, inducing the generation of new immature nerve cells that actively synthesize DNA. Indicators of DNA synthesis include the active uptake of exogenously administered nucleic acid analogues such as BrdU and the expression of markers of immature neurons such as double cortin. The effects of promoting nerve cell repair or inducing neurogenesis by the agent of the present invention can be evaluated, e.g., as mentioned in the Examples described below, by administering the agent to model animals that develop dementia or brain pathology and then observing the induction of brain-derived neurotrophic factor (BDNF) expression in the animals or by staining and observing brain sections of the animals.

According to an embodiment, the agent of the present invention is for removing a dementia-causing protein that accumulates in the brain. The term "dementia-causing protein" herein may refer to, although is not limited to, a protein that has been identified or presumed to be a cause of dementia. Examples include, although is not limited to, one or more proteins selected from amyloid β (Aβ), tau, α-synuclein, TDP-43, FUS/TLS, polyglutamine, protein resulting from RAN (repeat-associated non-ATG) translation, prion, and SOD-1. The effects of promoting nerve cell repair or removing a dementia-causing protein by the agent of the present invention can be evaluated, e.g., as mentioned in the Examples described below, by administering the agent to model animals that develop dementia and then staining and observing brain sections of the animals.

According to an embodiment, the agent of the present invention is for treating or preventing a neurodegenerative disease. The term "neurodegenerative disease" herein may refer to, although is not limited to, a neurodegenerative disease involving accumulation of any of the dementia-causing proteins mentioned above in the brain. Examples of neurodegenerative diseases include, although is not limited to, Alzheimer's disease (A$\beta$ and tau), frontotemporal lobar degeneration (tau, TDP-43, and FUS/TLS), Lewy body dementia ($\alpha$-synuclein), Parkinson's disease ($\alpha$-synuclein), multisystem atrophy ($\alpha$-synuclein), Huntington's disease (polyglutamine), amyotrophic lateral sclerosis (TDP-43, FUS/TLS, RAN protein, and SOD-1), spinal cord cerebellum degeneration (RAN protein), and Creutzfeldt-Jakob disease (prion) (examples of dementia-causing proteins that accumulate in each neurodegenerative disease are shown in parentheses). Among these, the agent for treating or preventing a neurodegenerative disease of the present invention may preferably be an agent for treating or preventing degenerative dementia. Examples of degenerative dementia include, but are not limited to, Alzheimer's disease, frontotemporal dementia, and Lewy body dementia. Among these, frontotemporal dementia is characterized by accumulation of tau and exemplified by Pick's disease, corticobasal degeneration, and progressive supranuclear palsy. The effect of treating or preventing a neurodegenerative disease by the agent of the present invention can be evaluated, e.g., as mentioned in the Examples described below, by administering the agent to model animals that develop a neurodegenerative disease (e.g., degenerative dementia) and then subjecting the animals to a cognitive function test such as the Morris water maze test and a motor function test such as the rotarod test, or by staining and observing brain sections of the animals.

[Form, use, and dosage of the agents]

The form of the agent of the present invention is not particularly limited. For example, an herbal medicine selected from jujube seed and Japanese sweet flag stem can be used as it is in any form, such as a crushed product, an extract of crushed product, or an extract residue of crushed product, or in combination with other ingredients such as desired excipients and/or carriers. When used in the form of food (the food product of the present invention) or pharmaceutical (the pharmaceutical product of the present invention) containing the agent of the present invention, the jujube seed and/or Japanese sweet flag stem, which is/are the active ingredient(s) of the agent of the present invention, can be mixed with other ingredients appropriate for the form of food or pharmaceutical. Details will be described later.

The method of use of the agent of the present invention is not particularly limited, but it may usually be administered orally. In particular, the agent of the present invention may preferably be used in the form of food (the food product of the present invention) or oral dug (the pharmaceutical product of the present invention). Details will be described later.

The dosage of the agent of the present invention is not particularly limited. For example, the agent of the present invention can be administered to the subject in an amount such that the dosage of the active ingredient, i.e., jujube seed and/or Japanese sweet flag stem, may usually be 0.05 mg/day or more, preferably 0.5 mg/day or more, and more preferably 1.0 mg/day or more, and usually 10 g/day or less, preferably 5 g/day or less, and further preferably 1 g/day or less.

[Food product]

An aspect of the present invention, a food product containing an agent of the present invention (the food product of the present invention) is provided. The food product of the present invention is characterized by containing any one of the agents of the present invention and is used for the purpose of the agent.

The food product of the present invention can be in any form that can be ingested orally, such as a solution, suspension, emulsion, powder, or solid molded product. In addition, as with the pharmaceuticals of the present invention described below, it can be made into dosage forms such as capsules, lozenges, syrups, and granules.

The food product of the present invention can be manufactured as, e.g., beverages such as green tea, black tea, coffee, soft drinks, alcoholic beverages, carbonated beverages, milk beverages, fruit juice beverages, nutritional drinks, concentrated beverages, powdered beverages (powdered juice, powdered soup, etc.); supplements; confectioneries such as candy, gummy candy, gum, chocolate, cookies, biscuits; frozen desserts such as ice cream; dairy products such as yogurt and processed milk; flour products such as cereals, bread, and cake mix; noodles such as soba; processed oil and fat products such as mayonnaise, whipped cream, and salad dressing; processed marine products; processed livestock products; and processed agricultural products. The food product of the present invention can be produced by adding the agent of the present invention during the production of these food products.

In addition to other food ingredients, the food product of the present invention may contain, as needed, various additives such as: sweeteners, colorants, preservatives, thickening agents, stabilizers, gelling agents or pasting agents, antioxidants such as ascorbic acid, color developers, bleaching agents, mold inhibitors or fungicides, yeast food, gum bases, alkaline agents, bittering agents, enzymes, glossing agents, flavorings, acidulants, chewing gum softeners, seasonings, tofu coagulants, emulsifiers, pH adjusters, leavening agents, vitamins, minerals, amino acids, and other nutritional fortifiers, as well as manufacturing aids.

The food product of the present invention may be provided as a food product that indicates the action, effects, functions, or uses of the agent of the present invention under the system of each country. For example, in Japan, the food products of the present invention may be manufactured as health functional foods (foods with specified health functions, foods with functional claims, and foods with nutritional functions).

The content of the active ingredients of the agent of the present invention, i.e., jujube seed and/or Japanese sweet flag stem, in the food product of the present invention can be set appropriately within the range in which the effect of the present invention can be obtained, as with the agent of the present invention. Specifically, the content of the active ingredients of the agent of the present invention, i.e., jujube seed and/or Japanese sweet flag stem, may be adjusted to withing the range of usually 0.1 mg/day or more, preferably 0.5 mg/day or more, more preferably 1.0 mg/day or more, and usually 10 g/day or less, preferably 5 g/day or less, more preferably 1 g/day or less.

The number of times and frequency of intake of the food product of the present invention are arbitrary and can be set as appropriate at any number of times and frequency, such as once to several times a day, every day, every other day, every two days, or once to seven times a week. By combining the desired amount of jujube seed and/or Japanese sweet flag stem with food in accordance with the desired number of times and frequency of intake, it is possible to provide the food product of the present invention that can be expected to have the desired effect.

The food product of the present invention is characterized by containing the agent of the present invention, and is extremely useful since it shares various advantages of the agent of the present invention. In addition, as it is a food product, it can be taken safely and conveniently not only by patients suffering from specific diseases but also by healthy people.

In addition, the food product of the present invention can be used not only for humans but also for animals other than humans to which the agent of the present invention can be applied.

[Pharmaceutical product]

An aspect of the present invention provides a pharmaceutical containing the agent of the present invention (the pharmaceutical product of the present invention). The pharmaceutical product of the present invention is characterized by containing any one of the agents of the present invention and is used for the purpose of the agent.

The route of administration of the pharmaceutical product of the present invention is not limited, but it may usually be an oral medicine (oral agent) administered orally.

The pharmaceutical product of the present invention can be manufactured in any dosage form by adding the agent of the present invention as an active ingredient. Examples of such dosage forms include: a capsule preparation, such as a tablet, soft capsule, or hard capsule; a solid preparation, such as a powder, granule, drop, or pill; a semi-solid preparation, such as a jelly; a liquid preparation, such as a syrup, suspension, or internal liquid. In this case, the pharmaceutical product of the present invention can be formulated as a pharmaceutical composition combining the agent of the present invention with other additives commonly used in the manufacture of oral agents, using pharmaceutical manufacturing methods known to those skilled in the art.

Examples of additives that can be used in the manufacture of the pharmaceutical product of the present invention include excipients, disintegrants, binders, lubricants, coating agents, dispersants, fluidizing agents, stabilizers, preservatives, buffering agents, masking agents, suspending agents, emulsifiers, fragrances, solubility aids, colorants, and thickening agents. The agent of the present invention may also be combined with a pharmaceutically acceptable carrier to provide the pharmaceutical product of the present invention with further enhanced action and effects.

The pharmaceutical product of the present invention may be a product that is deemed to be equivalent to a pharmaceutical under the legal system of each country. Examples include quasi-drugs in Japan.

The content of the active ingredients of the agent of the present invention, i.e., jujube seed and/or Japanese sweet flag stem, in the pharmaceutical product of the present invention can be set appropriately within the range in which the effect of the present invention can be obtained, as with the agent of the present invention. Specifically, the content of the active ingredients of the agent of the present invention, i.e., jujube seed and/or Japanese sweet flag stem, may be adjusted to withing the range of usually 0.1 mg/day or more, preferably 0.5 mg/day or more, more preferably 1.0 mg/day or more, and usually 10 g/day or less, preferably 5 g/day or less, more preferably 1 g/day or less.

The number of times and frequency of administration of the pharmaceutical product of the present invention are arbitrary and can be set as appropriate at any number of times and frequency, such as once to several times a day, every day, every other day, every two days, or once to seven times a week. By using the desired amount of jujube seed and/or Japanese sweet flag stem in accordance with the desired number of times and frequency of administration, it is possible to provide the pharmaceutical product of the present invention that can be expected to have the desired effect.

The pharmaceutical product of the present invention is characterized by containing the agent of the present invention, and is extremely useful since it shares various advantages of the agent of the present invention.

In addition, the pharmaceutical product of the present invention can be used not only for humans but also for animals other than humans to which the agent of the present invention can be applied.

[Method]

An aspect of the present invention provides a method for maintaining or improving a cerebral function, a method for promoting nerve cell repair or inducing neurogenesis, a method for removing dementia-causing protein that accumulates in the brain, and a method for treating or preventing a neurodegenerative disease (the method of the present invention), the method including administering to a subject one or more selected from an herbal medicine selected from jujube seed and a stem portion of Japanese sweet flag, the agent of the present invention, the food product of the present invention, and the pharmaceutical product of the present invention.

The method of the present invention can be carried out by administering to the subject jujube seed and/or Japanese sweet flag stem, which is an active ingredient of the agent of the present invention, in the form of the agent of the present invention, the food product of the present invention, and/or the pharmaceutical product of the present invention. The details of the method of the present invention are the same as those explained above for the agent of the present invention, the food product of the present invention, and the pharmaceutical product of the present invention.

**EXAMPLES**

The present invention will be described in more detail below with reference to specific Examples. However, these Examples are merely provided for the purpose of explanation, and the present invention is not limited to these Examples in any way.

[Materials and methods]

*Preparation of hot water extract, ethanol extract, extraction residue, and crushed product of jujube seed *(Ziziphi Spinosae Semen;* ZSS) and Japanese sweet flag stem *(Acori Graminei Rhizoma* stem; AGS) as well as hot water extract of Japanese sweet flag root *(Acori Graminei Rhizoma* root; AGR)

The hot water extract of jujube seed (Ext-ZSS) in Example Group A was prepared as follows. One kilogram of dried jujube seeds (ZSS) (origin: Hebei Province, China) was crushed, to which 10 kg of water was added, and the mixture was boiled for one hour to carry out hot water extraction. The mixture was then filtered, the filtrate was concentrated and heated for sterilization, to which 40 g of dextrin was added, and the mixture was dried to obtain 200 g of jujube seed extract.

The ethanol extract (EtOH-ext-Z) of jujube seed in Example Group A was prepared as follows. Crushed jujube seed (200 g) was added to 50% ethanol/water (900 g), and the mixture was boiled for one hour to carry out hot water extraction, after which the mixture was filtered to obtain a filtrate. 50% ethanol/water (900 kg) was added to the remaining residue, and hot water extraction and filtration were carried out under the same conditions. The filtrates were combined, concentrated, and heated for sterilization (80°C, 30 minutes), filtered through a 30-mesh filter, and freeze-dried to obtain ethanol/water extract powder of non-crushed jujube seeds (24.5 g).

The extract residue of jujube seed (Res-ZSS) in Example A3 and the crushed powder of jujube seed (Cru-ZSS) in Examples A3, A5, and A6 were prepared as follows. 5.3 kg of dried jujube seeds obtained from Auropure Life Science Co, Ltd. (Zhuzhou, Hunan, China) were sterilized (115°C, 1.5 hours), finely crushed using a hammer mill, and passed through a 3 mm screen to prepare 2.1 kg of coarse powder. The coarse powder (2.1 kg) was sieved through a vibrating screen with a mesh size of 500 $\mu$m to yield 1.3 kg of dried powder. Water (140 ml) was added to the dried jujube seed powder (10 g), and the mixture was heated with hot water at 90-95°C for 3 hours to carry out hot water extraction. The mixture was then filtered, and the residue was dried under reduced pressure at 40°C overnight to obtain the extraction residue (8.29 g).

The hot water extract of Japanese sweet flag root (Ext-AGR) and the hot water extract of Japanese sweet flag stem (Ext-AGS) in Example B1 were prepared as follows. 500 g of dried roots and 400 g of dried stems of Japanese sweet flag (Acorus gramineus Solander; origin: Hubei Province, China) were cut into small pieces, to which 8 times and 30 times their volumes of water, respectively, were added. Each mixture was heated at 90°C for 1 hour, and then filtered through a 32-mesh and subsequently a 200-mesh filter. The filtrate was collected, and the residue was further extracted by heating twice. The filtrates of the three extractions were combined, concentrated, and freeze-dried to obtain a viscous liquid extract. The yield of the hot water extract of Japanese sweet flag stem (Ext-AGS) was 130 g, and the solid content was 50.0% by weight. The yield of the hot water extract of Japanese sweet flag root (Ext-AGR) was 98 g, and the solid content was 52.2% by weight.

The extract residue (Res-AGS) and crushed powder (Cru-AGS) of Japanese sweet flag stem in Example B3 were prepared as follows. Dried stems of Japanese sweet flag (5.3 kg) were coarsely crushed using a cutter mill and passed through a 2 mm screen to prepare 5.1 kg of coarsely crushed product, which was then sterilized (170°C, 3 seconds) and finely crushed with a ball mill. The finely crushed product was then sieved through a vibrating screen with a mesh size of 500 $\mu$m to yield 4.0 kg of dried crushed powder. Water (700 ml) was added to the dried crushed Japanese sweet flag stem powder (50 g), and the mixture was heated at 90-95°C for 3 hours to carry out hot water extraction. The mixture was then filtered, and the residue was dried under reduced pressure at 40°C overnight to obtain the extraction residue (43.41 g).

*Component analysis of jujube seed (ZSS)

The component analysis of jujube seed (ZSS) was entrusted to the Japan Food Research Laboratories (Tokyo, Japan). Jujube seed (ZSS) is known to contain jujuboside A, jujuboside B, and spinosin as main components (Non-Patent Literature 27: Liu et al., Talanta, (2007), 71:668-675; and Non-Patent Literature 28: Wang et al., J. Pharm. Anal., (2019), 9 [6]:406-413). 0.2 g of crushed jujube seed powder was suspended in 30 mL of 50% ethanol and shaken for 10 minutes. After centrifugation, the supernatant was collected, and the pellet was further extracted with ethanol twice more. The supernatants obtained from the three extractions were combined to obtain a total of 100 mL of jujube seed (ZSS) extract.

The detection of jujuboside A and jujuboside B was carried out by diluting the jujube seed extract (ZSS) 10 times and subjecting the diluted sample to high-performance liquid chromatography (HPLC) separation using a reverse-phase InertSustain C18 column (GL Sciences, Tokyo, Japan) with a mobile phase consisting of a mixture of 0.1% formic acid and acetonitrile (63:37). Each of the separated fractions was analyzed sequentially using electrospray ionization mass spectrometry (ESI-MS) with a Xevo TQ MS (Waters Corporation, Milford, MA).

The detection of spinosin was carried out by subjecting the jujube seed extract (ZSS) to high-performance liquid chromatography (HPLC) separation using a reverse-phase Unison UK-C18 column (Imtakt USA, Portland, Oregon, USA) with a mobile phase consisting of a mixture of 0.1% formic acid and methanol (65:35). The absorbance at 270 nm was measured for each of the separated fractions.

*Mice

Six different neurodegenerative mouse models were used.

The APP23 mice are AD model mice expressing human APP with the Swedish (KM670/671NL) mutation (Non-Patent Literature 29: Stalder et al., Am. J. Pathol., (1999), 154[6]:1673-1684). These mice exhibit memory impairment at three months of age. According to previous studies by the present inventors, these mice exhibit accumulation of $A\beta$ oligomers, loss of synapses, and amyloid deposition at 15 months of age.

The Tau784 mice are FTD model mice that expresses both three-repetition and four-repetition human tau at adult age due to the presence of a tau intron mutation (with four-repetition human tau being dominantly expressed) (Non-Patent Literature 30: Umeda et al., Am. J. Clin. Pathol., (2013), 183[1]:211-225). According to previous studies by the present inventors, these mice exhibit tau hyperphosphorylation, tau oligomer formation, synapse loss, and memory impairment at 6 months of age, microglia activation at 12 months of age, and neurofibrillary changes and neuron loss at 15 months of age due to the unbalanced expression of tau isoforms.

The Hu$\alpha$-Syn (A53T) strain G2-3 mice were originally created as a PD model expressing human $\alpha$-synuclein with the A53T mutation (Non-Patent Literature 31: Rota et al., Transl. Neurodegener., (2019), 8:5). According to previous studies by the present inventors, these mice exhibit accumulation of $\alpha$-synuclein oligomers from four months after birth, cognitive impairment at six months after birth, and motor dysfunction at nine months after birth. Therefore, they can be considered a model of DLB until nine months after birth.

The APP-OSK mice are transgenic (Tg) mice expressing the Osaka mutant human APP, developed as an AD model that does not form amyloid plaques but induces amyloid $\beta$ oligomers (Non-Patent Literature 32: Tomiyama et al., J. Neurosci., (2010), 30[14]:4845-4856).

The OSK-K1 mice are knock-in mice with the Osaka mutation introduced into the mouse APP gene and was created as an AD model (Non-Patent Literature 33: Umeda et al., Acta Neuropathol. Commun., (2017), 5:59).

The C9-500 mice are FTD/ALS model mice in which human full-length C9orf72 gene harboring ~500 repeats of the GGGGCC sequence was introduced (Non-Patent Literature 36: Liu et al., Neuron., (2016), 90(3):521-34). According to previous studies by the present inventors, accumulation of phosphorylated TDP-43 was observed at 3 months of age, and synaptic loss, neuronal cell loss, and activation of microglia were observed at 6 months of age (Non-Patent Literature 37: Hatanaka Y et al., Biomedicines., (2022), 10(5):1080). Cognitive function begins to decline at 4.5 months postnatal, but motor function remains normal until 12 months postnatal. Therefore, this mouse model can be considered an FTD-TDP model until 12 months postnatal. These transgenic mice (Tg) are crossed with wild-type FVB/N Jc1 mice to maintain heterozygous transgenes.

All these transgenic (Tg) mice were maintained and used as heterozygous animals. All animal experiments were approved by the Ethics Committee of Osaka Metropolitan University (Osaka, Japan) and conducted in accordance with the Animal Experiment Guide of Osaka Metropolitan University.

*Treatment of mice

To compare the effects of the hot water extract of Japanese sweet flag stem (Ext-AGS) and the hot water extract of Japanese sweet flag root (Ext-AGR), each extract (solid content approx. 50% by weight) was diluted with water to 2.5 mg/mL to prepare a suspension (400 $\mu$L, i.e., 0.5 mg of solid content), which was orally administered to male and female Tau784 mice five times a week (Monday to Friday) for one month. As a control, the same amount of water was administered to age-matched Tg and non-Tg littermates.

To investigate the effects of jujube seed (ZSS) and Japanese sweet flag stem (AGS), hot water extract (Ext-ZSS), crushed powder (Cru-ZSS), and extract residue (Res-ZSS) of jujube seed and hot water extract (Ext-AGS), crushed powder (Cru-AGS), and extract residue (Res-AGS) of Japanese sweet flag stem were each suspended in water to a concentration of 0.33 or 1.65 mg/mL by ultrasonic treatment. Each suspension (300 $\mu$L, i.e., 0.1 mg or 0.5 mg of powder) was orally administered to male and female Tau784, APP23, C9-500, and Hu$\alpha$-Syn (A53T) mice five times a week for one month.

To test the mixture of the main components of jujube seed (ZSS), jujuboside A, jujuboside B, and spinosin (all from Biosynth, Compton, Berkshire, UK) were dissolved in water at concentrations of 1.52 g/mL, 0.667 g/mL, and 2.33 g/mL, respectively, to prepare a mixed solution. A 300 $\mu$L dose of the mixed solution was administered to Tau784 mice for one month. The dosage of each component was equivalent to the amount of each component in 0.5 mg of hot water extract of jujube seed (Ext-ZSS), i.e., 0.455 g of jujuboside A, 0.2 g of jujuboside B, and 0.7 g of spinosin.

To evaluate neurogenesis, 5-bromo-2'-deoxyuridine (BrdU; Sigma-Aldrich), a thymidine analog selectively incorporated into the DNA of proliferating cells, was dissolved in Tris-buffered saline (pH 7.6) to a concentration of 5 mg/mL. A 300 $\mu$L of the BrdU solution (i.e., 1.5 mg) was administered via intraperitoneal injection to the Hu$\alpha$-Syn (A53T) mice during the final five days of treatment with crushed powder.

*Behavior tests

The spatial reference memory of each mouse was evaluated using the Morris water maze test (Non-Patent Literature 34: Kelley Bromley-Brits et al., J. Vis. Exp., (2011), 53e2920). Specifically, a circular pool with a diameter of 1 m was used, and a platform with a diameter of 10 cm was placed 1 cm below the water surface at a location invisible to the mouse. The mouse was placed in the pool and allowed to swim for 60 seconds, and the time taken to reach the platform was measured (referred to as escape latency). If the mouse did not reach the platform within 60 seconds, the experimenter placed the mouse on the platform and allowed it to rest for a short period. This trial was conducted five times per day at five-minute intervals for four consecutive days for each mouse. This test procedure was used to measure memory acquisition ability.

The motor function of each mouse was evaluated using the rotarod test with a MK-610 rotarod treadmill (Muromachi Machinery, Tokyo). Specifically, the mouse was placed on a rod (rotating axis) rotating at 5 revolutions per minute and trained for 180 seconds to walk continuously without falling off. The mouse was then trained twice under the condition of gradually increasing the rotation speed from 4 rotations per minute to 40 rotations per minute over 240 seconds. The following day, the same accelerated rotarod test was performed twice at one-hour intervals under the same conditions. The time the mouse fell off the rod was measured, and the average of the two trials was calculated.

*Histological analysis of neuropathology

Following the behavior tests, the mice from each group were divided into two subgroups, one for histological analysis and the other for subsequent biochemical analysis. Brain sections were prepared according to previously reported methods (Non-Patent Literature 32: Tomiyama et al., J. Neurosci., (2010), 30[14]:4845-4856). Phosphorylated tau and tau oligomers were stained using mouse monoclonal AT8 antibody (Thermo Scientific, Waltham, Massachusetts, USA) and rabbit polyclonal T22 antibody (Sigma-Aldrich), respectively. A$\beta$ oligomers and amyloid deposits were stained using mouse monoclonal 11A1 antibody (IBL, Fujio, Japan) and rabbit polyclonal $\beta$001 antibody (Non-Patent Literature 35: Lippa et al., Arch Neurol., (1999), 56:1111-1118), respectively. Phosphorylated $\alpha$-synuclein and $\alpha$-synuclein oligomers were stained with rabbit monoclonal anti-$\alpha$-synuclein (Phospho S129) antibody (EP1536Y; Abcam, Cambridge, UK) and rabbit polyclonal Syn33 antibody (Sigma-Aldrich), respectively. Synaptophysin was stained using a mouse monoclonal antibody against synaptophysin (SVP-38; Sigma-Aldrich). TDP-43 was stained using a mouse monoclonal antibody against phosphorylated TDP-43 (pSer409/410-TDP-43; Cosmo Bio). The staining intensity or positive areas in specific brain regions were quantified using NIH Image J software.

*Histological analysis of BDNF expression and neurogenesis

BDNF expression was evaluated in Tau784 mice administered the crushed powder of jujube seed and Japanese sweet flag stem (Cru-ZSS and Cru-AGS). Brain sections were stained with mouse monoclonal anti-BDNF antibody (#9; DSHB, Iowa City, Iowa). The staining intensity in specific brain regions was quantified using NIH Image J software.

Neurogenesis was evaluated in Huα-Syn (A53T) mice administered the crushed powder of jujube seed (Cru-ZSS) and Japanese sweet flag stem (Cru-AGS). Brain sections were double-stained with mouse monoclonal anti-BrdU (IBL) and rabbit polyclonal anti-double cortin antibody (Abcam). Cells positive for both BrdU and double cortin in the stationary brain region were counted as newly generated neurons.

*Statistics analysis

Comparisons of averages between three or more groups was carried out by ANOVA or two-factor repeated measures ANOVA (for behavioral tests), followed by Fisher's PLSD test. A p-value < 0.05 was considered to indicate a significant difference.

[Example Group A: Study on jujube seed (ZSS)]

*Example A1: Study on the effects of hot water extract of jujube seed (Ext-ZSS) on Tau784 mice

The effects of jujube seed (ZSS) on Tau784 mice were investigated. Tau784 mice (14 months old, average body weight 35.1 g) were orally administered the hot water extract of jujube seed (Ext-ZSS) at 0.1 and 0.5 mg/day for one month.

Figure 1 shows the results of the Morris water maze test of Tau784 mice administered the hot water extract of jujube seed (Ext-ZSS) at 0.1mg per day and 0.5mg per day, in comparison with Tau784 mice administered water and non-genetically modified mice. The memory function of the mice improved in a dose-dependent manner. Specifically, high doses resulted in complete recovery to levels similar to those of non-Tg littermates, while low doses showed only moderate effects.

Figure 2 shows tau pathology in the hippocampal CA2/3 region of Tau784 mice administered the hot water extract of jujube seed (Ext-ZSS) at 0.1mg per day and 0.5mg per day, in comparison with Tau784 mice administered water and non-genetically modified mice. Specifically, Figure 2A indicates photographs of the staining results of phosphorylated tau and tau oligomers, and Figure 2B indicates graphs showing quantitative values of staining intensity in each photograph. According to the results of the tau pathology evaluation in the hippocampus using immunohistochemistry, the levels of phosphorylated tau and tau oligomers decreased significantly in a dose-dependent manner.

Figure 3 shows synaptophysin pathology in mossy fibers of the hippocampal CA2/3 region of Tau784 mice administered the hot water extract of jujube seed (Ext-ZSS) at 0.1mg per day and 0.5mg per day, in comparison with Tau784 mice administered water and non-genetically modified mice. Specifically, Figure 3A indicates photographs of the staining results of synaptophysin, and Figure 3B indicates graphs showing quantitative values of staining intensity in each photograph. Synaptophysin levels in the CA2/3 region of the hippocampus also recovered significantly in a dose-dependent manner.

*Example A2: Study on the effects of hot water extract of jujube seed (Ext-ZSS) on APP23 mice

The effects of jujube seed (ZSS) on APP23 mice were investigated. APP23 mice aged 13 to 15 months (average body weight 28.9 g) were orally administered the hot water extract of jujube seed (Ext-ZSS) at 0.1 mg/day for one month. In addition, 15- to 16-month-old APP23 mice (average body weight 28.9 g) were orally administered the hot water extract of jujube seed (Ext-ZSS) at 0.5 mg/day for one month.

Figure 4 indicates graphs showing the results of the Morris water maze test of APP23 mice administered the hot water extract of jujube seed (Ext-ZSS), in comparison with APP23 mice administered water and non-genetically modified mice. Specifically, Figure 4A is a graph showing the results of administration at 0.1 mg/day, and Figure 4B is a graph showing the results of administration at 0.5 mg/day. The hot water extract of jujube seed (Ext-ZSS) improved memory in mice at all doses. In particular, administration of the hot water extract of jujube seed (Ext-ZSS) at 0.5 mg/day significantly improved the memory function of the mice to levels similar to those of the non-Tg littermates.

Figure 5 shows amyloid β oligomer pathology in the cerebral cortex (CC) and hippocampus (HC) of APP23 mice administered the hot water extract of jujube seed (Ext-ZSS) at 0.1mg per day, in comparison with APP23 mice administered water. Specifically, Figure 5A indicates photographs of the staining results of amyloid β oligomers, and Figure 5B indicates graphs showing quantitative values of staining intensity in each photograph. The hot water extract of jujube seed (Ext-ZSS) considerably reduced the levels of Aβ oligomers in the cerebral cortex and hippocampus.

Figure 6 shows amyloid deposition pathology in the cerebral cortex (CC) and hippocampus (HC) of APP23 mice administered the hot water extract of jujube seed (Ext-ZSS) at 0.1mg per day, in comparison with APP23 mice administered water. Specifically, Figure 6A indicates photographs of the staining results of amyloid deposition, and Figure 6B indicates graphs showing quantitative values of staining intensity in each photograph. The hot water extract of jujube seed (Ext-ZSS) significantly reduced the levels of Aβ oligomers both in the cerebral cortex and hippocampus.

Figure 7 shows synaptophysin pathology in the hippocampal CA2/3 region of APP23 mice administered the hot water extract of jujube seed (Ext-ZSS) at 0.1mg per day, in comparison with APP23 mice administered water and non-genetically modified mice. Specifically, Figure 7A indicates photographs of the staining results of synaptophysin, and Figure 7B indicates graphs showing quantitative values of staining intensity in each photograph. The hot water extract of jujube seed (Ext-ZSS) significantly reduced the levels of synaptophysin level in the hippocampal CA2/3 region.

*Example A3: Study on the effects of hot water extract (Ext-ZSS), crushed powder (Cru-ZSS), and extract residue (Res-ZSS) of jujube seed on Tau784 mice

In traditional Chinese "Kampo" herbal medicine, in most cases, herbs are typically used as decoctions, and the residue from the extraction process is usually discarded. However, in hot water extraction, heat-sensitive components may break down, and volatile substances may be lost through evaporation. Therefore, to investigate whether there is a difference in efficacy depending on the forms of jujube seed, an experiment was carried out to examine the effects of the hot water extract (Ext-ZSS), the crushed powder (Cru-ZSS), and the extract residue (Res-ZSS) on Tau784 mice.

Specifically, the hot water extract (Ext-ZSS) and the crushed powder (Cru-ZSS) of jujube seed were administered to Tau784 mice (average body weight 29.3 g) aged 8 to 12 months at 0.1 mg/day for one month for comparison. In addition, the crushed powder (Cr-ZSS) and the extraction residue (Res-ZSS) of jujube seed were administered to Tau784 mice (average body weight 31.2 g) aged 8 to 11 months at 0.1 mg/day for one month for comparison.

Figure 8 indicates graphs showing the results of the Morris water maze test Tau784 mice administered the hot water extract (Ext-ZSS), the crushed powder (Cru-ZSS), and the extraction residue (Res-ZSS) of jujube seed at 0.1mg per day, in comparison with Tau784 mice administered water and non-genetically modified mice. Figure 8A is a graph showing the results of comparison between the hot water extract (Ext-ZSS) and the crushed powder (Cru-ZSS). The hot water extract (Ext-ZSS) improved the memory of the mice, but the effect was not sufficient. On the other hand, the crushed powder (Cru-ZSS) significantly enhanced the memory of the mice to levels even higher than those of the non-Tg littermates. Figure 8B is a graph showing the results of comparison between the extraction residue (Res-ZSS) and the crushed powder (Cru-ZSS). In this evaluation, the crushed powder (Cr-ZSS) also showed extremely good memory-improving effects. On the other hand, the hot water extract (Ext-ZSS) also improved the memory of the mice as with the extraction residue (Res-ZSS), but the effects were insufficient. These results support the present inventors' assumption that the functional components may be lost when the herb is used in the form of decoction.

Figure 9 shows tau pathology in the hippocampal CA2/3 region of Tau784 mice administered the hot water extract of jujube seed (Ext-ZSS), the crushed powder (Cru-ZSS), or the extraction residue (Res-ZSS) at 0.1mg per day, in comparison with Tau784 mice administered water and non-genetically modified mice. Figure 9A indicates photographs of the staining results of phosphorylated tau and tau oligomers, and Figure 9B indicates graphs showing quantitative values of staining intensity in each photograph. The hot water extract (Ext-ZSS), crushed powder (Cru-ZSS), and extraction residue (Res-ZSS) considerably decreased the levels of phosphorylated tau and tau oligomers, and the crushed powder (Cru-ZSS) showed the strongest effect.

Figure 10 shows synaptophysin and BDNF pathology in the hippocampal CA2/3 region of Tau784 mice administered the hot water extract (Ext-ZSS), the crushed powder (Cru-ZSS), or the extraction residue (Res-ZSS) of jujube seed at 0.1mg per day, in comparison with Tau784 mice administered water and non-genetically modified mice. Figure 10A indicates photographs of the staining results of synaptophysin and BDNF, and Figure 10B indicates graphs showing quantitative values of staining intensity in each photograph. The crushed powder (Cru-ZSS) significantly restored the synaptophysin level in the hippocampal CA2/3 region to a level similar to the level of the non-Tg littermates, whereas the hot water extract (Ext-ZSS) and the extraction residue (Res-ZSS) showed only a slight effect. The crushed powder (Cru-ZSS) also significantly restored the expression level of BDNF in the hippocampus to a level higher than that of the non-Tg littermates, whereas the hot water extract (Ext-ZSS) and the extract residue (Res-ZSS) showed only a slight effect.

*Example A4: Study of the effects of jujuboside A, jujuboside B, and spinosin, the main components of jujube seed (ZSS), on Tau784 mice

Jujuboside A, jujuboside B, and spinosin, which are main components of jujube seed (ZSS), have all been reported to have anti-dementia effects. Accordingly, it was speculated that the reason why the crushed jujube seed powder (Cru-ZSS) showed a stronger effect against tau than the hot water extract (Ext-ZSS) was because crushed powder (Cru-ZSS) contained more of these components than hot water extract (Ext-ZSS is because the crushed powder (Cru-ZSS) contained these main components at larger amounts than those in the hot water extract (Ext-ZSS).

To verify this possibility, the amounts of these three components in the hot water extract (Ext-ZSS) and the crushed powder (Cru-ZSS) of jujube seed were analyzed. The results are shown in Table 1 below. Unexpectedly, despite its strong dementia-suppressing effect, the crushed powder (Cr-ZSS) contained lower amounts of these components than those in the hot water extract (Ex-ZSS). These results suggest that the effect of the crushed jujube seed powder (Cr-ZSS) is due to components other than these components.

[Table 1]

Table 1. Contents of main components in 100 g of jujube seed (ZSS) preparations

| Component | Hot water extract | Crushed powder |
|---|---|---|
| Jujuboside A | 91mg | 44mg |
| Jujuboside B | 40mg | 31mg |
| Spinosin | 140mg | 68mg |

Therefore, in order to evaluate the contribution of jujuboside A, jujuboside B, and spinosin to the improvement of cognitive function in mice, these compounds were dissolved in water and mixed to prepare a mixture solution having the final content of each component corresponding to 0.5 mg of the hot water extract of jujube seed (Ext-ZSS), i.e., 0.455 $\mu$g of jujuboside A, 0.2 $\mu$g of jujuboside B, and 0.7 $\mu$g of spinosin in 300 $\mu$L, and administered it to Tau784 mice (average body weight 31.9 g) aged 13-16 months for one month.

Figure 11 is a graph showing the results of the Morris water maze test of Tau784 mice administered a mixed solution of the three main components of jujube seed, i.e., jujuboside A, jujuboside B, and spinosi, in comparison with Tau784 mice administered water and non-genetically modified mice. The daily doses of jujuboside A, jujuboside B, and spinicin in the mixed solution were 0.455 $\mu$g, 0.2 $\mu$g, and 0.7 $\mu$g, respectively, which correspond to the amounts of each component in 0.5 mg of the hot water extract of jujube seed (Ext-ZSS). The effect of the mixture solution of jujuboside A, jujuboside B, and spinosin on the memory of the mice was much weaker than that of 0.5 mg of the hot water extract of jujube seed (Ext-ZSS) (Figure 1). This result suggests that jujube seed (ZSS) contains active substances other than jujuboside A, jujuboside B, and spinosin, and that most of these substances may be lost during hot water extraction.

*Example A5: Study on the effects of the crushed jujube seed powder (Cru-ZSS) on Hu-$\alpha$-Syn (A53T) mice

The effects of jujube seed (ZSS) were investigated in the $\alpha$-synucleinopathy model. 8-month-old Hu$\alpha$-Syn (A53T) mice (average body weight, 29.8 g) were orally administered the crushed jujube seed powder (Cru-ZSS) at 0.1 mg/day for one month. Immunohistochemistry was used to evaluate $\alpha$-synuclein pathology in the hippocampus. In addition, since the crushed jujube seed powder (Cru-ZSS) was suggested to have a nutritional effect on neurons (Figures 8 to 10), the levels of neurogenesis in the dentate gyrus and substantia nigra were evaluated. The latter brain region is particularly vulnerable to neurodegeneration induced by $\alpha$-synuclein and may cause motor dysfunction in PD.

Figure 12 shows $\alpha$-synuclein pathology in the hippocampal CA2/3 region of Hu$\alpha$-Syn (A53T) mice administered the crushed powder of jujube seed (Cru-ZSS) at 0.1mg per day, in comparison with Tau784 mice administered water and non-genetically modified mice. Specifically, Figure 12A indicates photographs of the staining results of phosphorylated $\alpha$-synuclein and $\alpha$-synuclein oligomers, and Figure 12B indicates graphs showing quantitative values of staining intensity in each photograph. The crushed powder of jujube seed (Cr-ZSS) significantly reduced the levels of phosphorylated $\alpha$-synuclein and $\alpha$-synuclein oligomers.

Figure 13 shows the neurogenesis levels in the dentate gyrus (DG) and substantia nigra (SN) of Hu$\alpha$-Syn (A53T) mice administered the crushed powder of jujube seed (Cru-ZSS) at 0.1mg per day, in comparison with Hu$\alpha$-Syn (A53T) mice administered water and non-genetically modified mice. Specifically, Figure 13A indicates photographs of immunofluorescence staining results of BrdU (red) and doublecortin (DCX, green), and Figure 13B indicates graphs showing the results of counting double-positive cells (yellow) in each photograph as newborn neurons. The crushed powder of jujube seed (Cru-ZSS) significantly increased the number of BrdU- and double cortin-positive cells in all brain regions to levels higher than those in the non-Tg littermates.

*Example A6: Study on the effects of the crushed jujube seed powder (Cru-ZSS) on Hu-A-Syn (A53T) mice

The effects of jujube seed (ZSS) on the motor function of mice were investigated. 8-month-old Hu$\alpha$-Syn (A53T) mice (average body weight, ZSS 29.8 g) were orally administered the crushed jujube seed powder (Cru-ZSS) at 0.1 mg/day for one month. Subsequently, motor function was evaluated using the rotarod test.

Figure 14 is a graph showing the result of the rotarod test of Hu$\alpha$-Syn (A53T) mice administered the crushed powder of jujube seed (Cru-ZSS) at 0.1mg per day in Example A6, in comparison with Hu$\alpha$-Syn (A53T) mice administered water and non-genetically modified mice. The crushed powder of jujube seed (Cru-ZSS) significantly increased the motor function of the Hu$\alpha$-Syn (A53T) mice to a level similar to that of the non-Tg littermates.

*Example A7: Comparison of the effects of the hot water extract (HOT water ext-Z) and the ethanol extract (EtOH ext-Z) of jujube seed on APPOSK mice

The effects of the hot water extract and the ethanol extract of crushed jujube seed on APP/OSK mice were investigated. Ten APP/OSK mice aged 12 to 16 months were divided into three groups. One group was administered 0.1 mg/day of the hot water extract of crushed jujube seed, another group was administered 0.1 mg/day of the ethanol extract of crushed jujube seed, and the remaining group was administered 300 μL of water orally for five days a week for a total of one month. Non-Tg mice of the same age were administered the same amount of water orally.

Figure 15 is a graph showing the results of the Morris water maze test of APPOSK mice administered the hot water extract of jujube seed (HOT water ext-Z) or the ethanol extract (EtOH ext-Z), in comparison with APPOSK mice administered water and non-genetically modified mice. As long as crushed jujube seed was used, there was no difference in medicinal efficacy between the two extracts, and both extracts were equally effective.

*Example A8: Comparison of crushed and uncrushed jujube seeds

Four groups of nine OSK-KI mice aged 14 to 16 months were divided into four groups. The first group was administered 0.1 mg/day of the hot water extract of crushed jujube seed (Crushed-HOT water ext-Z), the second group was administered 0.1 mg/day of the hot water extract of non-crushed jujube seeds (Intact-HOT water ext-Z), the third group was administered 0.1 mg/day of ethanol extract of non-crushed jujube seeds (Intact-EtOH ext-Z), and the fourth group was administered water, each at 300μL on 5 days a week for a total of one month. Non-KI mice of the same age were administered the same amount of water orally.

Figure 16 is a graph showing the results of the Morris water maze test of OSK-KI mice administered the hot water extract of crushed jujube seed (Crushed-HOT water ext-Z), the hot water extract of non-crushed jujube seeds (Intact-HOT water ext-Z), or the ethanol extract of non-crushed jujube seeds (Intact-EtOH ext-Z), in comparison with OSK-KI mice administered water and non-genetically modified mice. Compared with the hot water extract of crushed jujube seed, the hot water extract and the ethanol extract of non-crushed jujube seeds were both slightly less effective. No difference was observed between the hot water extract and the ethanol extract of non-crushed jujube seeds.

[Example Group B: Study on Japanese sweet flag stem (AGS)]

*Example B1: Study on the effects of the hot water extract (Ext-AGS) and the hot water extract (Ext-AGR) of Japanese sweet flag stem on Tau784 mice

The effects of Japanese sweet flag stem (AGS) and Japanese sweet flag root (AGR) were compared in Tau784 mice. The former is used in traditional Chinese medicine, while the latter is usually discarded. Tau784 mice (average weight 36.1 g) aged 17 months were orally administered the hot water extracts of Japanese sweet flag stem (Ext-AGS) and Japanese sweet flag root (Ext-AGR) each containing 0.5 mg of solid matter for one month.

Figure 17 is a graph showing the results of the Morris water maze test of Tau784 mice administered the hot water extract of Japanese sweet flag stem (Ext-AGS) or the hot water extract of Japanese sweet flag root (Ext-AGR) at 0.5mg per day, in comparison with Tau784 mice administered water and non-genetically modified mice. In the water maze test, both extracts significantly improved the memory of mice, but unexpectedly, AGS showed a stronger effect than AGR.

Figure 18 shows tau pathology in the hippocampal CA2/3 region of Tau784 mice administered the hot water extract of Japanese sweet flag stem (Ext-AGS) or the hot water extract of Japanese sweet flag root (Ext-AGR) at 0.5mg per day, in comparison with Tau784 mice administered water and non-genetically modified mice. Specifically, Figure 18A indicates photographs of the staining results of phosphorylated tau and tau oligomers, and Figure 18B indicates graphs showing quantitative values of staining intensity in each photograph. According to the results of tau pathology evaluation of the olfactory cortex using immunohistochemistry, both extracts significantly reduced the level of phosphorylated tau and also reduced the level of tau oligomers, but Japanese sweet flag stem (AGS) showed a stronger effect than Japanese sweet flag root (AGR).

Figure 19 shows synaptophysin pathology in mossy fibers of the hippocampal CA2/3 region of Tau784 mice administered the hot water extract of Japanese sweet flag stem (Ext-AGS) or the hot water extract of Japanese sweet flag root (Ext-AGR) at 0.5mg per day, in comparison with Tau784 mice administered water and non-genetically modified mice. Specifically, Figure 19A indicates photographs of the staining results of synaptophysin, and Figure 19B indicates graphs showing quantitative values of staining intensity in each photograph. Both extracts restored the level of synaptophysin in the hippocampal CA3 region to the same level as that of the non-Tg littermates. To the best of the knowledge of the present inventors, these results are the first evidence that AGS has an effect equivalent to or greater than that of AGR in improving cognitive function.

*Example B2: Study on the effects of hot water extract of Japanese sweet flag stem (Ext-AGS) on APP23 mice

The effects of Japanese sweet flag stem (AGS) on APP23 mice were investigated. APP23 mice (average weight 28.9 g) aged 13 to 15 months were orally administered 0.1 mg/day of the hot water extract of Japanese sweet flag stem (Ext-AGS) for one month.

Figure 20 is a graph showing the results of the Morris water maze test of APP23 mice administered the hot water extract of Japanese sweet flag stem (Ext-AGS) at 0.1mg per day, in comparison with APP23 mice administered water and non-genetically modified mice. The hot water extract of Japanese sweet flag stem (Ext-AGS) improved the memory of the mice at a dosage of 0.1mg per day.

Figure 21 shows amyloid $\beta$ oligomer pathology in the cerebral cortex (CC) and hippocampus (HC) of APP23 mice administered the hot water extract of Japanese sweet flag stem (Ext-AGS) at 0.1mg per day, in comparison with APP23 mice administered water. Figure 21A indicates photographs of the staining results of amyloid $\beta$ oligomers, and Figure 21B indicates graphs showing quantitative values of staining intensity in each photograph. The hot water extract of Japanese sweet flag stem (Ext-AGS) considerably reduced the levels of A$\beta$ oligomers in the cerebral cortex and hippocampus.

Figure 22 shows amyloid deposition pathology in the cerebral cortex (CC) and hippocampus (HC) of APP23 mice administered the hot water extract of Japanese sweet flag stem (Ext-AGS) at 0.1mg per day, in comparison with APP23 mice administered water. Specifically, Figure 22A indicates photographs of the staining results of amyloid deposition, and Figure 22B indicates graphs showing quantitative values of staining intensity in each photograph. The hot water extract of Japanese sweet flag stem (Ext-AGS) significantly decreased the amyloid depositions both in the cerebral cortex (CC) and hippocampus (HC).

Figure 23 shows synaptophysin pathology in the hippocampal CA2/3 region of APP23 mice administered the hot water extract of Japanese sweet flag stem (Ext-AGS) at 0.1mg per day, in comparison with APP23 mice administered water and non-genetically modified mice. Specifically, Figure 23A indicates photographs of the staining results of synaptophysin, and Figure 23B indicates graphs showing quantitative values of staining intensity in each photograph. hot water extract of Japanese sweet flag stem (Ext-AGS) significantly decreased the synaptophysin level of the hippocampal CA2/3 region.

*Example B3: Study on the effects of the hot water extract (Ext-AGS), the crushed powder (Cru-AGS), and the extraction residue (Res-AGS) of Japanese sweet flag stem on Tau784 mice

In traditional Chinese "Kampo" herbal medicine, in most cases, herbs are typically used as decoctions, and the residue from the extraction process is usually discarded. However, in hot water extraction, heat-sensitive components may break down, and volatile substances may be lost through evaporation. Therefore, to investigate whether there is a difference in efficacy depending on the forms of Japanese sweet flag stem, an experiment was carried out to examine the effects of the hot water extract (Ext-AGS), crushed powder (Cru-AGS), or extraction residue (Res-AGS) of Japanese sweet flag stem on Tau784 mice.

Specifically, the hot water extract (Ext-AGS), the crushed powder (Cru-AGS), or the extraction residue (Res-AGS) of Japanese sweet flag stem was administered to 10-month-old Tau784 mice (average body weight 29.3g) at 0.1mg per day for a month for comparison.

Figure 24 indicates graphs showing the results of the Morris water maze test Tau784 mice administered the hot water extract (Ext-AGS), the crushed powder (Cru-AGS), or the extraction residue (Res-AGS) of Japanese sweet flag stem at 0.1mg per day, in comparison with Tau784 mice administered water and non-genetically modified mice. Specifically, Figure 24A is a graph showing the results of the hot water extract (Ext-AGS) and the crushed powder (Cru-AGS). The hot water extract (Ext-AGS) improved the memory of the mice, but its effect was not sufficient. On the other hand, the crushed powder (Cru-AGS) enhanced the memory of the mice to the same level as that of the non-Tg littermates. Figure 24B is a graph showing the results of the extraction residue (Res-AGS) and the crushed powder (Cru-AGS). The crushed powder (Cru-AGS) achieved complete recovery, while the extraction residue (Res-AGS) showed only moderate effects. These results support the present inventors' hypothesis that the functional components may be lost when the herb is used in the form of decoction.

Figure 25 shows tau pathology in the hippocampal CA2/3 region of Tau784 mice administered the hot water extract (Ext-AGS), the crushed powder (Cru-AGS), or the extraction residue (Res-AGS) of Japanese sweet flag stem at 0.1mg per day, in comparison with Tau784 mice administered water and non-genetically modified mice. Specifically, Figure 25A indicates photographs of the staining results of phosphorylated tau and tau oligomers, and Figure 25B indicates graphs showing quantitative values of staining intensity in each photograph. The crushed powder (Cru-AGS) considerably decreased the level of phosphorylated tau, while the hot water extract (Ext-AGS) or the extraction residue (Res-AGS) only achieved a slight decrease. All three preparations decreased the level of tau oligomers significantly, with the crushed powder (Cru-AGS) showing the strongest effect.

Figure 26 shows synaptophysin and BDNF pathology in the hippocampal CA2/3 region of Tau784 mice administered the hot water extract (Ext-AGS), crushed powder (Cru-AGS), or extraction residue (Res-AGS) of Japanese sweet flag stem at 0.1mg per day, in comparison with Tau784 mice administered water and non-genetically modified mice. Specifically, Figure 26A indicates photographs of the staining results of synaptophysin and BDNF, and Figure 26B indicates graphs showing quantitative values of staining intensity in each photograph. All three preparations considerably decreased the level of synaptophysin in the hippocampal CA2/3 region, but only the crushed powder (Cru-AGS) restored the level to the same level as that of the non-Tg littermates (Figure 5 D). Although the BDNF levels in the hippocampus were lower than those in the non-Tg littermates, they increased considerably with the crushed powder (Cru-AGS) and slightly with the hot water extract (Ext-AGS) and the extract residue (Res-AGS).

*Example B4: Study on the effects of the crushed powder of Japanese sweet flag stem (Cru-AGS) on Huα-Syn (A53T) mice

The effects of Japanese sweet flag stem (AGS) on the α-synucleinopathy model were investigated. Eight-month-old Huα-Syn (A53T) mice (average body weight, AGS 29.0 g) were orally administered the crushed Japanese sweet flag stem powder (Cru-AGS) at 0.1 mg/day for one month. The pathology of α-synuclein in the hippocampus was evaluated by immunohistochemistry. In addition, since the crushed Japanese sweet flag stem powder (Cru-AGS) was suggested to have a nutritional effect on neurons (Figures 24 to 26), the levels of neurogenesis in the dentate gyrus and substantia nigra were evaluated. The latter brain region is particularly vulnerable to α-synuclein-induced neurodegeneration and may cause motor dysfunction in PD.

Figure 27 shows α-synuclein pathology in the hippocampal CA2/3 region of Huα-Syn (A53T) mice administered the crushed powder of Japanese sweet flag stem (Cru-AGS) at 0.1mg per day, in comparison with Tau784 mice administered water and non-genetically modified mice. Specifically, Figure 27A indicates photographs of the staining results of phosphorylated α-synuclein and α-synuclein oligomers, and Figure 27B indicates graphs showing quantitative values of staining intensity in each photograph. The crushed powder of Japanese sweet flag stem (Cru-AGS) increased the levels of phosphorylated α-synuclein and α-synuclein oligomers considerably.

Figure 28 shows the neurogenesis levels in the dentate gyrus (DG) and substantia nigra (SN) of Huα-Syn (A53T) mice administered the crushed powder of Japanese sweet flag stem (Cru-AGS) at 0.1mg per day, in comparison with Huα-Syn (A53T) mice administered water and non-genetically modified mice. Specifically, Figure 28A indicates photographs of immunofluorescence staining results of BrdU (red) and doublecortin (DCX, green), and Figure 28B indicates graphs showing the results of counting double-positive cells (yellow) in each photograph as newborn neurons. The crushed powder of Japanese sweet flag stem (Cru-AGS) restored the neurogenerative levels in the dentate gyrus and substantia nigra to levels similar to those of the non-Tg littermates.

[Example Group C: Study comparing the effects of Japanese sweet flag stem (AGS) and jujube seed (ZSS)]

The effects of Japanese sweet flag stem (AGS) and jujube seed (ZSS) on C9-500 mice (Jackson Laboratory) were compared. Seven-month-old C9-500 mice (average weight 31 g) were orally administered a water suspension (0.33 mg/mL) containing 0.1 mg of crushed Japanese sweet flag stem (Cru-AGS) or jujube seed (Cru-ZSS) for one month.

*Example C1: Comparative study of the effects of Japanese sweet flag stem (AGS) and jujube seed (ZSS) on C9-500 mice

Figure 29 is a graph showing the results of the Morris water maze test of C9-500 mice administered the crushed product of Japanese sweet flag stem (Cru-AGS) or the crushed product of jujube seed (Cru-ZSS) at 0.1mg per day. In the water maze test, both extracts significantly improved the memory of mice, but unexpectedly, the Japanese sweet flag stem (AGS) showed a stronger effect than the jujube seed (ZSS).

Figure 30 shows TDP-43 pathology in the hippocampal CA2/3 region of C9-500 mice administered the crushed product of Japanese sweet flag stem (Cru-AGS) or the crushed product of jujube seed (Cru-ZSS) at 0.1mg per day, in comparison with C9-500 mice administered water and non-genetically modified mice. Specifically, Figure 30A indicates photographs of the staining results of phosphorylated TDP-43, and Figure 30B indicates graphs showing quantitative values of puncta in each photograph. Both crushed products decreased the level of TDP-43, but the Japanese sweet flag stem (AGS) showed a stronger effect than that of the jujube seed (ZSS).

Figure 31 shows synaptophysin pathology in mossy fibers of the hippocampal CA2/3 region of C9-500 mice administered the crushed product of Japanese sweet flag stem (Cru-AGS) or crushed product of jujube seed (Cru-ZSS) at 0.1mg per day, in comparison with C9-500 mice administered water and non-genetically modified mice. Specifically, Figure 31A indicates photographs of the staining results of synaptophysin, and Figure 31B indicates graphs showing quantitative values of staining intensity in each photograph. The crushed product of Japanese sweet flag stem (AGS) significantly increased the level of synaptophysin in the hippocampus CA3region.

*Example C2: Comparative study of the effects of Japanese sweet flag stem (AGS) and jujube seed (ZSS) on HuαSyn mice

The effect of Japanese sweet flag stem (AGS) on the α-synucleinopathy model was investigated. 6- to 7-month old Huα-Syn (A53T) mice (average body weight: AGS 28.0g) were orally administered the crushed powder of Japanese sweet flag stem (Cru-AGS) or the crushed powder of jujube seed (Cru-ZSS) at 0.1mg per day for a month, and then subjected to the Morris water maze test. The results are shown in Figure 32. In the water maze test, both extracts improved the memory of the mice considerably, but unexpectedly, the Japanese sweet flag stem (AGS) showed a stronger effect than that of the jujube seed (ZSS).

[Example Group D: Study on methods for crushing jujube seeds (ZSS)]

*Example D1: Study on the crushing and preparation method of jujube seed (ZSS)

Dried jujube seeds obtained from Auropure Life Science Co, Ltd. (Zhuzhou, Hunan, China) in an amount of 8.6 kg were flash roasted at 260°C for 57 seconds, then processed in an ultra-fine grinding machine (Masukuroider, Zouko Sangyo Co., Ltd., #MKZA10-10JLC αH) and sieved through a 2.38 mm mesh to obtain coarse powder (8.49 kg). 100 g of the obtained coarse powder (8.49 kg) was subjected to airflow crushing (Micro Powtech Co., Ltd., # MP10-550) and then sieved through a 0.5 mm mesh to obtain a fine powder (64 g).

The particle size distributions of a crushed powder of jujube seed (ZSS) obtained by the airflow crushing method and a crushed powder of jujube seed (ZSS) obtained by the hammer mill crushing method (used in Examples A3, A5, and A6) were analyzed. Particle size analysis was performed using a laser diffraction and scattering particle size analyzer (LMS-3000, Malvern) by circulating 150 mL of isopropanol and adding one microspatulaful of circulating jujube seed powder to the dispersion tank. Ultrasonic treatment was performed while monitoring the light diffraction and scattering data of the sample particles, and the particle size distribution was measured once the dispersion state became uniform. The measurement range was set to 0.01-3,500.00 μm. The results are shown in Figure 33. The vertical axis of Figure 33 represents the volume (%) obtained by dividing the scattering intensity of each particle size by the total scattering intensity of all particle sizes. As shown in Figure 33, the hammer mill crushing method detected peak tops at 1 μm, 6 μm, and 300-400 μm, while the airflow crushing method detected peak tops at 1 μm, 6 μm, 20-30 μm, and 100 μm. As shown in the cumulative volume ratio, particles with a particle size of 100 μm or less accounted for 50% or less in the hammer mill crushing method, whereas particles with a particle size of 100 μm or less accounted for 75% or more in the airflow crushing method. The crushed powder obtained by the airflow crushing method has a finer overall particle size, resulting in a smooth texture without clumping.

Furthermore, the physical properties of the crushed powders of jujube seed (ZSS) obtained by the hammer mill crushing and airflow crushing methods were analyzed. The results are shown in Table 2.

[Table 2]

Table 2: Physical properties of crushed powders of jujube seed (ZSS) obtained by the hammer mill crushing and airflow crushing methods

|  | Repose angle | Water activity | Bulk density | Acid value | Peroxide value |
|---|---|---|---|---|---|
| Hammer mill crushing | 51° | 0.63 | 0.66 | 13.5 | 1 |
| Airflow crushing | 48° | 0.32 | 0.52 | 8.7 | 5 |

(Repose angle)

The granules were deposited horizontally from the orifice (spout) to form a cone. The angle between the generatrix of the cone and the base was calculated as the repose angle α using the radius r of the base and the height h of the cone using the following equation.

[Formula 1]

$$\alpha = \tan^{-1}\left(\frac{h}{r}\right)$$

(Bulk density)

Measurements were taken using a standard method with a measuring cylinder. The sample was passed through a sieve with a mesh size of 1.0 mm or more, and 100 g of the sample was placed in a 250 mL measurement cylinder and leveled without pressing down. The loose bulk volume V0 was measured and used for calculating the bulk density g/mL

(Water activity)

A 20 mm diameter aluminum dish (its mass is denoted as $W_0$ (g)) was used to place 1.0 to 1.1 g of the test sample. The sample and saturated solution were then placed into the Conway unit. The aluminum dish containing the sample was placed into the inner chamber of the Conway unit, and the standard saturated solution (selected from Table 3 below; its mass is denoted as $W_1$ (g)) was placed into the outer chamber. After sealing, the unit was incubated at 25°C for 2 hours. After the treatment, the aluminum dish was weighed (its mass is denoted as $W_2$ (g)). The increase or decrease in mass per 1 g of the sample was plotted on the Y-axis, and the water activity of the saturated solution was plotted on the X-axis. The intersection point with the X-axis was calsulated and used as the water activity of the sample.

[Formula 2]

$$(\text{Increase or decrease per 1 g of the sample}) = \frac{W_2 - W_1 - W_0}{W_1}$$

[Table 3]

Table 3: Water activity of saturated solution (25°C)

| Reagent | Water activity | Reagent | Water activity |
|---|---|---|---|
| $K_2Cr_2O_7$ | 0.980 | $SrCl_2 \cdot 6H_2O$ | 0.708 |
| $KNO_3$ | 0.924 | NaBr | 0.576 |
| $BaCl_2*2H_2O$ | 0.901 | $Mg(NO_3)_2 \cdot 6H_2O$ | 0.528 |
| KCl | 0.842 | $K_2CO_3$ | 0.432 |
| KBr | 0.807 | $MgCl \cdot 6H_2O$ | 0.330 |
| NaCl | 0.752 | $LiCl \cdot H_2O$ | 0.112 |
| $NaNO_3$ | 0.737 | | |

(Acid value)

Fifteen grams of the sample were combined with 200 mL of diethyl ether to extract oil components. The mixture was then dehydrated and filtered, and the solvent was removed to obtain the extracted oil, which was dissolved in 60 mL of a 1:1 mixture of ethanol and diethyl ether. A glass electrode GE-101B was connected to COM-1760 manufactured by Heiwa Sangyo Co., Ltd. The potential difference was measured by titrating with a 0.05 mol/L potassium hydroxide standard solution.

(Peroxide value)

Fifteen grams of the sample were combined with 200 mL of diethyl ether to extract the oil components. The mixture was then dehydrated and filtered, and the solvent was removed to obtain the extracted oil, which was dissolved in 50 mL of a 2:3 mixture of isooctane and acetic acid for nitrogen substitution, and then combined with 0.1 mL of saturated potassium iodide. After reacting for 1 minute, 30 mL of water was added and allowed to permeate. The resulting solution was titrated with a 0.01 mol/L sodium thiosulfate standard solution using a 1% starch solution as an indicator.

The powder prepared by autoclave sterilization and hammer mill crushing and the powder treated by airflow crushing after sterilization by roasting were both found to have a particle size distribution of 0.5 mm or less by particle size distribution analysis. This is similar to that of general starch (repose angle 45-58°: "Powder Particle Precision Supply Technology" (Japanese textbook), Riki Shibata, IPC Co., Ltd.) and soybean flour (51°: "Powder Particle Precision Supply Technology" (Japanese textbook), Riki Shibata, IPC Co., Ltd.) and wheat flour (53-56°: "Powder Particle Precision Supply Technology" (Japanese textbook), Riki Shibata, IPC Co., Ltd.) and soybean flour (51°: "Powder Particle Precision Supply Technology" (Japanese textbook), Riki Shibata, IPC Co., Ltd.), and wheat flour (53-56°: Powder and Granule Precision Supply Technology, by Riki Shibata, IPC Co., Ltd.). The repose angle was also found to be comparable to that of general wheat flour (0.56-0.72: "Powder Particle Precision Supply Technology" (Japanese textbook), Riki Shibata, IPC Co., Ltd.) and sugar powder (0.66: "Powder Particle Precision Supply Technology" (Japanese textbook), Riki Shibata, IPC Co., Ltd.) and soybean flour (0.66: "Powder Particle Precision Supply Technology" (Japanese textbook), Riki Shibata, IPC Co., Ltd.). In addition, while the peroxide value indicated that there was almost no oxidation, the acid value suggested that there was a high content of free fatty acids. Of particular note is the water activity. Both the hammer mill crushing method and the airflow crushing method have a water activity of less than 0.7, which is considered to be highly preservable, and the airflow crushing method has a water activity of less than 0.5, which is sufficient to inhibit microbial growth. Based on the above, both the hammer mill crushing and airflow crushing methods have excellent preservability in terms of bacterial growth inhibition, etc., and can be expected to be applied in industries that include food processing similar to wheat flour, which has a water activity of 0.61 to 0.63 (according to the Ministry of Health, Labour and Welfare's "Handbook for Hygiene Management Incorporating HACCP Concepts in Wheat Flour Manufacturing," Japanese document).

Jujube seeds were crushed four times in 50 g batches using a mill (Russell Hobbs, #7660JP) without sterilization to obtain a simple crushed product. The nutritional components of the crushed jujube seed powder obtained by the simple crushing method, hammer mill crushing method, and airflow crushing method were analyzed. The results are shown in Table 4 below.

[Table 4]

| Table 4: Nutritional components of crushed jujube seeds obtained by each crushing method | | | |
|---|---|---|---|
| | Simple crushing (/100g) | Hummer mill crushing (/100g) | Airflow crushing (/100g) |
| Moisture content | 9.6g | 7.2 g | 2.3 g |
| Proteins | 35.4g | 34.9 g | 37.9 g |
| Lipids | 25.6g | 25.6 g | 28.0 g |
| Ash | 2.9g | 2.8 g | 3.1 g |
| Carbohydrates | 26.5g | 29.5 g | 28.7 g |
| Sugars | 3.2g | 2.4 g | 3.0 g |
| Dietary fiber | 23.3g | 27.1 g | 25.7 g |
| Energy | 431kcal | 434 kcal | 467 kcal |
| Sodium | <1.0mg | 1.1 mg | <1.0mg |
| Salt equivalent | <0.0050 g | 0.0028 g | <0.0050 g |

(Moisture content)

A sample weighing 3.9 to 4.1 g (designated as S (g)) was placed in an aluminum weighing tube (its mass is denoted as $W_1$ (g)), which was incubated at 70°C for 5 hours in a vacuum drying oven. After cooling in a silica gel desiccator, the total mass of the weighing tube and sample (designated as $W_2$ (g)) was measured, and the moisture content was calculated using the following formula.

[Formula 3]

$$\left(Moisture\ content\ \left(\frac{g}{100g\ sample}\right)\right) = \frac{W_1 + S - W_2}{S}$$

(Proteins)

A sample weighing 0.3 to 0.4 g was placed in quartz and analyzed using a total nitrogen analyzer (manufactured by Sumika Analytical Center Co., Ltd.) with high-purity helium gas (purity 99.99% or higher) as the carrier gas and high-purity oxygen gas (purity 99.99% or higher) as the oxidizing gas. The analysis was conducted with the following settings: reaction furnace temperature of 870°C or higher, reduction furnace temperature of 600°C, detector temperature of 100°C, and column temperature of 70°C. The calibration curve was prepared using ethylenediamine tetraacetic acid (EDTA) as the standard. The protein mass was calculated from the nitrogen mass obtained using the following equation. Note that N is the nitrogen mass (mg) calculated from the calibration curve, S is the sample mass (g), and K is the nitrogen-to-protein conversion factor of 6.25.

[Formula 4]

$$\left(Total\ nitrogen\ mass\ \left(\frac{g}{100g\ sample}\right)\right) = \frac{\frac{N}{1000}}{S} \times 100$$

[Formula 5]

$$\left(Protein\ mass\ \left(\frac{g}{100g\ sample}\right)\right) = (Total\ nitrogen\ mass\ ) \times K$$

(Lipids)

A sample weighing 0.5 to 1.4 g (Sg) was combined with 2 mL of ethanol. Hydrochloric acid and pure water were mixed in a ratio of 25:11, and 10 mL of the mixture was added to the sample mixture, which was then decomposed at a temperature of 70-80°C in a constant-temperature water bath for 30-40 minutes. The resulting acid decomposition product was transferred to a Majonia tube, to which 10 mL of ethanol was added, followed by 25 mL of diethyl ether, and the mixture was shaken and mixed. 25 mL of petroleum ether was then added, and the mixture was shaken and mixed. The ether mixture phase (designated as "E1") was separated from the aqueous phase, and 40 mL of a mixture of diethyl ether and petroleum ether (equal volumes) was added to the aqueous phase, shaken and mixed. The ether mixture phase (designated as "E2") was separated from the aqueous phase, and 30 mL of a mixture of diethyl ether and petroleum ether (equal volumes) was added to the aqueous phase and mixed. The ether mixture phase (hereinafter referred to as "E3") was separated from the aqueous phase, which was removed. The obtained ether mixture phases E1, E2, and E3 were combined and placed in a weighed fat bottle ($W_1$ g), which was then subjected to removal of the solvent and dried at 105°C for 1 hour. The residue was then cooled in a silica gel desiccator, then weigh (this is denoted as $W_2$ (g)).

[Formula 6]

$$\left(lipid\ mass\ \left(\frac{g}{100gsample}\right)\right) = \frac{W_2 - W_1}{S} \times 100$$

(Ash)

A magnetic crucible was weighed (its mass is denoted as W1 (g)), and a sample of 1.0 to 1.2 g (mass denoted as S (g)) was placed in the crucible and weighed. After preliminary ash fusion, the sample was ash-fused at 550°C, cooled in a silica gel desiccator, and weighed again (this mass is denoted as $W_2$ (g)). The ash content was calculated using the following equation.

[Formula 7]

$$\left(Ash\ mass\ \left(\frac{g}{100gsample}\right)\right) = \frac{W_2 - W_1}{S} \times 100$$

(Carbohydrates)

The carbohydrate content was calculated using the following formula in accordance with the Food Labeling Standards (Cabinet Office Ordinance No. 10 of 2015).

[Formula 8]

$$100 - (\text{Moisture content} + \text{Protein} + \text{Lipid} + \text{Ash})$$

(Sugars)

The sugar content was calculated using the following formula in accordance with the Food Labeling Standards (Cabinet Office Ordinance No. 10 of 2015).

[Formula 9]

$$100 - (\text{Moisture content} + \text{Protein} + \text{Lipid} + \text{Ash} + \text{Dietary fiber})$$

(Dietary fiber)

Approximately 1 g of the sample was placed in a long-neck tube and degreased twice with petroleum ether. The sample was divided equally into two 500 mL tall beakers, to each of which 0.08 mL phosphate buffer solution (pH 6.0) was added to adjust the volume to 50 mL each. 0.05 mL of Termamyl (Novozymes) was then added to each mixture, which was incubated for 30 minutes with shaking in a boiling water bath. After cooling, the pH was adjusted to 4.3 ± 0.3 with approximately 10 mL of 0.325 mol/L hydrochloric acid, and each mixture was combined with 0.1 mL of amyloglucosidase (Sigma-Aldrich) and incubated at 60°C for 30 minutes with shaking. Each mixture was diluted fourfold with 95% ethanol at 60°C and allowed to stand at room temperature for 1 hour. Each mixture was then filtered through a 2G2 glass filter (filled with 1 g of celite) under suction, and the residue was washed three times with 20 mL of 78% ethanol, twice with 10 mL of 95% ethanol, twice with 10 mL of acetone, and once with 50 mL of petroleum ether. Each residue was then dried overnight at 105°C, weighed and denoted as R1 and R2. One of the two samples obtained was treated at 525°C for 5 hours to ash, weighed, and the ash mass was denoted as A. The remaining sample was used to determine the protein mass P using the nitrogen quantitative replacement method (coefficient 6.25). The dietary fiber mass was calculated using the following equation based on the above mass data. In the equation below, R is the average mass of the residue, P is the protein content (%) in the residue, A is the ash content (%) in the residue, S is the sample mass, r is the mass of the blank residue, p is the protein content (%) in the blank residue, and a is the ash content (%) in the blank residue.

[Formula 10]

$$Lipid\ mass\ \left(\frac{g}{100g\ sample}\right) = \frac{R \times \left(1 - \frac{P + A}{100}\right) - r \times \left(1 - \frac{p + a}{100}\right)}{S} \times 100$$

(Energy)

The energy was calculated as an energy conversion factor in accordance with the Food Labeling Standards (Cabinet Office Ordinance No. 10 of 2015), with protein at 4, fat at 9, carbohydrates at 4, and dietary fiber at 2.

(Sodium)

One gram of the sample was placed in a quartz beaker, calcified at 500°C for 10 hours, combined with 2.5 mL of 20% hydrochloric acid, and evaporated to dryness. 2.5 mL of 20% hydrochloric acid was added to the resulting sample, which was heat extracted, filtered (using filter paper No. 5A), and diluted to 50 mL. The solution was then measured using an atomic absorption spectrophotometer (SpectrAA240FS, Agilent Technologies) with a sodium cathode lamp (Agilent Technologies) as the light source, at a measurement wavelength of 589.0 nm, with a frame of acetylene at 2.00 L/min and air at 13.50 L/min.

The nutritional component data (moisture, protein, lipid, ash, carbohydrate, sugar, dietary fiber) of the powders prepared by the hammer mill crushing method and the airflow crushing method were standardized using the nutritional components of the powder prepared by the simple crushing method. The resultant data are shown in Table 5 below. The hammer mill crushing method tended to reduce the moisture and sugar contents, while increasing the carbohydrate and dietary fiber contents. The airflow crushing method dramatically reduced the moisture and sugar contents, while increasing the protein, lipid, ash, carbohydrate, and dietary fiber contents. As described below, since the powder prepared by the hammer mill crushing method tends to improve cognitive function in disease model mice more strongly than the powder prepared by the airflow crushing method, nutritional components present in higher amounts may contain an active pharmaceutical ingredient. This study suggests that dietary fiber may contain an active pharmaceutical ingredient. In addition, as mentioned above, since the pharmacological activity remained in both the hot water extract and the extract residue of jujube seed, it is possible that both water-soluble and insoluble dietary fiber may contain an active pharmaceutical ingredient.

[Table 5]

| Table 5: Nutritional components in powders prepared by hammer mill crushing and airflow crushing | | |
|---|---|---|
| Nutritional component | Hammer mill crushing | Airflow crushing |
| Moisture content | 75% | 24% |
| Proteins | 99% | 107% |
| Lipids | 100% | 109% |
| Ash | 97% | 107% |
| Carbohydrates | 111% | 108% |
| Sugars | 75% | 94% |
| Dietary fiber | 116% | 110% |
| *Standardized with each nutritional component content in the powder obtained by the simple crushing method | | |

(Salt equivalent)

Calculated by multiplying the sodium mass by 2.54.

*Example D2: Study on the effect of crushing and preparation methods

As in Example A3, the effects of the crushed powders of jujube seed (ZSS) prepared by different methods on Tau784 mice were examined. The results are shown in Figure 34, which is a graph showing the results of the Morris water maze test of Tau784 mice administered the crushed powder of jujube seed (Cru-ZSS new) prepared in Example D1 at 1 mg/kg/day or 3 mg/kg/day, in comparison with Tau784 mice administered water and non-genetically modified mice. The crushed powder of jujube seed obtained by the airflow crushing method also showed extremely excellent memory improvement effects.

**INDISUTIAL APPLICABILITY**

The present invention can be widely applied in fields such as functional foods and pharmaceuticals where maintenance or improvement of cognitive functions is required, and therefore has an extremely high utility value.

**Claims**

1.  An agent for maintaining or improving a cerebral function, the agent comprising an herbal medicine selected from jujube seed (*Ziziphi Spinosae Semen*) and a stem portion of Japanese sweet flag (*Acori Graminei/Tatarinowii Rhizoma*), wherein the jujube seed is in a form selected from a crushed product and an extraction residue of crushed product, and the stem portion of Japanese sweet flag is in a form selected from a crushed product, an extract of crushed product, and an extraction residue of crushed product.

2.  The agent for maintaining or improving a cerebral function according to claim 1, wherein the cerebral function is cognitive function.

3.  An agent for promoting nerve cell repair or inducing neurogenesis, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

4. An agent for removing a dementia-causing protein that accumulates in the brain, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

5. The agent according to claim 4, wherein the dementia-causing protein is one or more selected from amyloid β (Aβ), tau, α-synuclein, TDP-43, FUS/TLS, polyglutamine, protein resulting from RAN (repeat-associated non-ATG) translation, prion, and SOD-1.

6. An agent for treating or preventing a neurodegenerative disease, the agent comprising an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

7. The agent according to claim 6, wherein the neurodegenerative disease is neurodegenerative dementia.

8. The agent according to claim 7, wherein the neurodegenerative dementia is one or more selected from Alzheimer's disease, frontotemporal dementia, and Lewy body dementia.

9. The agent according to any one of claims 1 to 8, wherein the herbal medicine is administered to a subject at an amount of from 0.05mg to 10g per day.

10. A food product comprising the agent according to any one of claims 1 to 8.

11. A pharmaceutical product comprising the agent according to any one of claims 1 to 8.

12. A method for maintaining or improving a cerebral function in a subject, the method comprising administering to a subject in need thereof an herbal medicine selected from jujube seed (*Ziziphi Spinosae Semen*) and a stem portion of Japanese sweet flag (*Acori Graminei/Tatarinowii Rhizoma*), wherein the jujube seed is in a form selected from a crushed product and an extraction residue of crushed product, and the stem portion of Japanese sweet flag is in a form selected from a crushed product, an extract of crushed product, and an extraction residue of crushed product.

13. The method according to claim 12, wherein the cerebral function is cognitive function.

14. A method for promoting nerve cell repair or inducing neurogenesis in a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

15. A method for removing a dementia-causing protein that accumulates in the brain of a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

16. The method according to claim 15, wherein the dementia-causing protein is one or more selected from amyloid β (Aβ), tau, α-synuclein, TDP-43, FUS/TLS, polyglutamine, proteins resulting from RAN (repeat-associated non-ATG) translation, prion, and SOD-1.

17. A method for treating or preventing a neurodegenerative disease in a subject, the method comprising administering to a subject in need thereof an herbal medicine selected from jujube seed and a stem portion of Japanese sweet flag, wherein each of the jujube seed and the stem portion of Japanese sweet flag selected from, independently of each other, a crushed product, an extract of crushed product, and an extraction residue of crushed product.

18. The method according to claim 17, wherein the neurodegenerative disease is neurodegenerative dementia.

19. The method according to claim 18, wherein the neurodegenerative dementia is one or more selected from Alzheimer's disease, frontotemporal dementia, and Lewy body dementia.

20. The method according to any one of claims 12 to 19, wherein the herbal medicine is administered to the subject at an amount of from 0.05mg to 10g per day.

21. Crushed powder of an herbal medicine selected from jujube seed and a stem portion of Japanese sweet flag, wherein the percentage of crushed powder particles with a particle size of 100 $\mu$m or less to the total crushed powder particles is 50% or more.

22. The crushed powder according to claim 21, which has a moisture content activity of less than 0.7.

Fig. 1

Non-Tg (n=9)    Tg + Ext-ZSS (0.1 mg) (n=10)
Tg (n=9)    Tg + Ext-ZSS (0.5 mg) (n=10)

15mo Tau784

Fisher's PLSD
* Tg: $p$ = 0.0011 vs non-Tg
† Ext-ZSS (0.1 mg): $p$ = 0.0478 vs Tg
‡ Ext-ZSS (0.5 mg): $p$ = 0.0010 vs Tg

Fig. 2

A

B

Fig. 3

A

B

Fig. 4

A

B

Fig. 5

A

B

Fig. 6

A

B

Fig. 7

A

Synaptophysin

HC

Non-Tg　　　　　Tg　　　　Tg + Ext-ZSS

40 μm

B

Fig. 8

A

B

Fig. 9

A

B

Fig. 10

Fig. 11

Fig. 12

A

B

Fig. 13

A

B

Fig. 14

Fig. 15

Acquisition test

Legend:
- APPosk (n=11)
- Non-Tg (n=9)
- Hot water ext-Z, 0.1mg (n=10)
- EtOH ext-Z, 0.1mg (n=10)

Fisher's PLSD

\* $p = 0.0003$, non-Tg vs Tg
† $p = 0.0372$, non-Tg vs Tg+HW ext-Z (0.1mg)
‡ $p = 0.0365$, non-Tg vs Tg+EtOH ext-Z (0.1mg)

APPosk 13-17 mo

Fig. 16

Acquisition test

Legend:
- OSK-KI (n=9)
- Non-KI (n=9)
- Crushed-Hot water ext-Z, 0.1mg (n=9)
- Intact-Hot water ext-Z, 0.1mg (n=9)
- Intact-EtOH ext-Z, 0.1mg (n=9)

Fisher's PLSD

* $p = 0.0364$, non-KI vs KI
† $p = 0.0465$, KI vs KI+Crsh-HW ext-Z (0.1mg)

OSK-KI 15-17 mo

Fig. 17

Legend:
- Tau784 (n=8)
- Non-Tg (n=9)
- Tg + Ext-AGR (0.5 mg solid matter) (n=9)
- Tg + Ext-AGS (0.5 mg solid matter) (n=9)

Y-axis: Escape latency (sec)
X-axis: Day

18 mo Tau784

Fisher's PLSD

* Tg: $p = 0.0140$ vs non-Tg,
† Ext-AGR: $p = 0.0491$ vs Tg
‡ Ext-AGS: $p = 0.0064$ vs Tg

Fig. 18

A

B

Fig. 19

A

Synaptophysin

| Non-Tg | Tg | Tg + Ext-AGS | Tg + Ext-AGR |

30 µm

B

Fig. 20

Fig. 21

A

B

Fig. 22

A

Amyloid deposits

80 μm

B

Fig. 23

A

Synaptophysin

| Non-Tg | Tg | Tg + Ext-AGS |

HC

40 μm

B

Fig. 24

A

B

Fig. 25

Fig. 26

A

B

Fig. 27

A

B

Fig. 28

A

B

Fig. 29

Legend:
- C9-500 (n=11)
- FVB (n=10)
- Cru-ZSS, 100ug (n=10)
- Cru-AGS, 100ug (n=10)

Y-axis: Escape latency (sec)
X-axis: Day

7mon C9-500

Fisher's PLSD
* Tg: $p = 0.0003$ vs non-Tg
† ZSS-pwd: $p = 0.0372$ vs Tg
‡ AGS-pwd: $p = 0.0039$ vs Tg

Fig. 30

A

B

Fig. 31

A

B

Fig. 32

Fisher's PLSD
* Tg: *p* < 0.0001 vs non-Tg
† AGS-pwd: *p* = 0.0060 vs Tg
‡ ZSS-pwd: *p* = 0.0005 vs Tg

Fig. 33

A — Jujube seed, hammer mill crushing

B — Jujube seed, airflow crushing

Fig. 34

## INTERNATIONAL SEARCH REPORT

| International application No. |
|---|
| **PCT/JP2023/046811** |

| A. | CLASSIFICATION OF SUBJECT MATTER |
|---|---|

*A61K 36/725*(2006.01)i; *A23L 33/105*(2016.01)i; *A61K 36/882*(2006.01)i; *A61P 25/00*(2006.01)i; *A61P 25/28*(2006.01)i; *A61P 43/00*(2006.01)i

FI:    A61K36/725; A61K36/882; A61P25/00; A61P25/28; A61P43/00 121; A23L33/105

According to International Patent Classification (IPC) or to both national classification and IPC

| B. | FIELDS SEARCHED |
|---|---|

Minimum documentation searched (classification system followed by classification symbols)

A61K36/725; A23L33/105; A61K36/882; A61P25/00; A61P25/28; A61P43/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| X | 高久俊 他, 衛気虚証患者の慢性頭痛に対して玉屏風散(加酸棗仁)末が著効した2例, 日本東洋医学雑誌, 2017, vol. 68, 別冊号, page. 406, O-242. (TAKAKU, Shun et al., Kampo Medicine), non-official translation (Two cases in which Gyokuheifusan (added jujube seeds) powder was significantly effective against chronic headaches in Eiki deficiency patients, separate volume)<br>lines 11-12 | 10-11, 21-22 |
| X | 山田直明, 患者と私 112不眠, パニック障害, 漢方研究, 2014, no. 509, pages 161, 162, (YAMADA, Naoaki, Progress in Kampo Medicine), non-official translation (Patient and I 112 insomnia, panic disorder)<br>pages 161, 162, tables of [progress] | 10-11, 21-22 |
| X | CN 1765267 A (SONG, Min) 03 May 2006 (2006-05-03)<br>claim 3, page 1/4, lines 3, 4, page 2/4, lines 10, 11, page 4/4, example (1) | 21-22 |
| X | KR 10-2004-0023197 A (BACK, Il Sung) 18 March 2004 (2004-03-18)<br>example 11 | 21-22 |

| ✓ Further documents are listed in the continuation of Box C. | ✓ See patent family annex. |
|---|---|

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 March 2024** | **19 March 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

## INTERNATIONAL SEARCH REPORT

| | International application No. |
|---|---|
| | PCT/JP2023/046811 |

### C. DOCUMENTS CONSIDERED TO BE RELEVANT

| Category* | Citation of document, with indication, where appropriate, of the relevant passages | Relevant to claim No. |
|---|---|---|
| X | MORIYAMA, H. et al., Ameliorative effect of sansoninto on sleep disturbance and spatial memory impairment in an Alzheimer's disease rat model, Traditional & Kampo Medicine, 2017, vol. 4, no. 1, pages 38-45<br>abstract, fig. 4 | 1-9, 12-20 |
| X | JP 2015-500803 A (MINISTRY OF FOOD AND DRUG SAFETY) 08 January 2015 (2015-01-08)<br>claim 1, paragraphs [0015]-[0017], experimental examples 1-4 | 1-9, 12-20 |
| A | LEE, B. et al., Protective effects of methanol extract of Acori graminei rhizoma and Uncariae Ramulus et Uncus on ischemia-induced neuronal death and cognitive impairments in the rat, Life Sciences, 2003, vol. 74, no. 4, pages 435-450<br>abstract | 1-9, 12-20 |
| A | JP 2012-6906 A (KOREA INSTITUTE OF SCIENCE AND TECNOLOGY) 12 January 2012 (2012-01-12)<br>claims 1, 3, paragraph [0025] | 1-9, 12-20 |
| A | JP 2013-209354 A (KANPO IKAGAKU KENKYUSHO KK) 10 October 2013 (2013-10-10)<br>claim 1, paragraph [0028] | 1-9, 12-20 |
| P, A | 梅田知宙他, 認知症ゼロ社会に向けた認知症予防アンチエイジング食品の開発, Dementia Japan, October 2023, vol. 37, no. 4, page 711, P354, (UMEDA, Tomohiro et al.), non-official translation (Development of anti-aging food to prevent dementia towards a dementia-free society) | 1-22 |

Form PCT/ISA/210 (second sheet) (July 2022)

## INTERNATIONAL SEARCH REPORT
### Information on patent family members

International application No.

**PCT/JP2023/046811**

| Patent document cited in search report | | | Publication date (day/month/year) | Patent family member(s) | Publication date (day/month/year) |
|---|---|---|---|---|---|
| CN | 1765267 | A | 03 May 2006 | (Family: none) | |
| KR | 10-2004-0023197 | A | 18 March 2004 | (Family: none) | |
| JP | 2015-500803 | A | 08 January 2015 | US 2015/0065441 A1 claim 1, paragraphs [0024]-[0026], experimental examples 1-4 WO 2013/081419 A1 EP 2786758 A1 KR 10-2013-0060835 A CN 104105488 A | |
| JP | 2012-6906 | A | 12 January 2012 | US 2011/0318435 A1 claims 1, 3, paragraph [0030] KR 10-2011-0140015 A | |
| JP | 2013-209354 | A | 10 October 2013 | (Family: none) | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SPIRES-JONES et al.** *Acta Neuropathol.*, 2017, vol. 134 (2), 187-205
- **LUO et al.** *Cell & Biosci.*, 2022, vol. 12, 2
- **VALIUKAS et al.** *Vaccines*, 2022, vol. 10 (9), 1527
- **SONG et al.** *Transl. Neurodegener.*, 2022, vol. 11, 18
- **MEDINA**. *Int. J. Mol. Sci.*, 2018, vol. 19 (4), 1160
- **JI et al.** *Drugs*, 2021, vol. 81 (10), 1135-1152
- **ASHER et al.** *Life Sciences*, 2022, vol. 306, 120861
- *Chinese Traditional and Herbal Drugs*, 2001, vol. 32 (3), 246-247
- *Journal of Guangxi Traditional Chinese Medical University*, 2002, vol. 5 (3), 11-13
- *Journal of Xi'an Jiaotong University (Medical Sciences)*, 2010, vol. 31 (6), 673-707
- **LIU et al.** *Eur. J. Pharmacol.*, 2014, vol. 738, 206-213
- **ZHANG et al.** *Theranostics*, 2018, vol. 8, 4262-4278
- **TABASSUM et al.** *Sci. Rep.*, 2019, vol. 9, 4512
- **KO et al.** *Biomol. Ther.*, 2015, vol. 23, 156-164
- **LEE et al.** *Pharmacol. Biochem. Behav.*, 2016, vol. 145, 9-16
- **XU et al.** *Biomol. Ther.*, 2019, vol. 27, 71-77
- **CAI et al.** *Biomol. Ther.*, 2020, vol. 28, 131-136
- **ZHANG et al.** *Biomol. Ther.*, 2020, vol. 28, 259-266
- **GENG et al.** *Biol. Pharm. Bull.*, 2010, vol. 33, 836-843
- **LIU et al.** *Yakugaku Zasshi*, 2010, vol. 130 (5), 737-746
- **WEI et al.** *J. Alzheimers Dis.*, 2013, vol. 33, 863-880

- **YANG et al.** *Cell Mol. Neurobiol.*, 2016, vol. 36, 121-130
- **XUE et al.** *Eur. J. Pharmacol.*, 2014, vol. 741, 195-204
- **CHEN et al.** *Brain Res.*, 2014, vol. 1552, 41-54
- **MAO et al.** *Aging Cell*, 2015, vol. 14, 784-796
- **LIU et al.** *Talanta*, 2007, 71668-675
- **WANG et al.** *J. Pharm. Anal.*, 2019, vol. 9 (6), 406-413
- **STALDER et al.** *Am. J. Pathol.*, 1999, vol. 154 (6), 1673-1684
- **UMEDA et al.** *Am. J. Clin. Pathol.*, 2013, vol. 183 (1), 211-225
- **ROTA et al.** *Transl. Neurodegener.*, 2019, vol. 8, 5
- **TOMIYAMA et al.** *J. Neurosci.*, 2010, vol. 30 (14), 4845-4856
- **UMEDA et al.** *Acta Neuropathol. Commun.*, 2017, vol. 5, 59
- **KELLEY BROMLEY-BRITS et al.** *J. Vis. Exp.*, 2011, vol. 53, e2920
- **LIPPA et al.** *Arch Neurol.*, 1999, 561111-1118
- **LIU et al.** *Neuron.*, 2016, vol. 90 (3), 521-34
- **HATANAKA et al.** *Biomedicines*, 2022, vol. 10 (5), 1080
- Guidance and Regulation on Unapproved and Unauthorized Drugs. Ministry of Health and Welfare, 18 April 2018
- **LIU et al.** *Yakugaku Zasshi*, 2010, vol. 130, 737-746
- **LIU et al.** *Talanta*, 2007, vol. 71, 668-675
- **HATANAKA Y et al.** *Biomedicines*, 2022, vol. 10 (5), 1080
- **LIPPA et al.** *Arch Neurol.*, 1999, vol. 56, 1111-1118
- Handbook for Hygiene Management Incorporating HACCP Concepts in Wheat Flour Manufacturing. Ministry of Health, Labour and Welfare's